(19) Europäisches Patentamt
European Patent Office
Office européen des brevets

(11) EP 1 251 170 A2

(12) # EUROPEAN PATENT APPLICATION

(43) Date of publication:
23.10.2002 Bulletin 2002/43

(51) Int Cl.7: **C12N 15/11**, A61K 31/713, C12N 9/00

(21) Application number: **02005642.0**

(22) Date of filing: **02.07.1993**

(84) Designated Contracting States:
**AT BE CH DE DK ES FR GB GR IE IT LI LU MC NL PT SE**

(30) Priority: **17.07.1992 US 916763**
**07.12.1992 US 987132**
**07.12.1992 US 989849**
**07.12.1992 US 989848**
**19.01.1993 US 8895**

(62) Document number(s) of the earlier application(s) in accordance with Art. 76 EPC:
**93918144.2 / 0 654 077**

(71) Applicant: **RIBOZYME PHARMACEUTICALS, INC.**
**Boulder, CO 80301 (US)**

(72) Inventors:
- **Sullivan, Sean M.**
**Boulder, CO 80301 (US)**
- **Draper, Kenneth G.**
**Boulder, CO 80301 (US)**

(74) Representative: **Viering, Jentschura & Partner**
**Steinsdorfstrasse 6**
**80538 München (DE)**

Remarks:
This application was filed on 12 - 03 - 2002 as a divisional application to the application mentioned under INID code 62.

(54) **Method and reagent for treatment of NF-kappaB dependent animal diseases**

(57) An enzymatic RNA molecule which cleaves mRNA associated with development or maintenance of an inflammatory disease, an arthritic condition, a stenotic condition, or a cardiovascular condition.

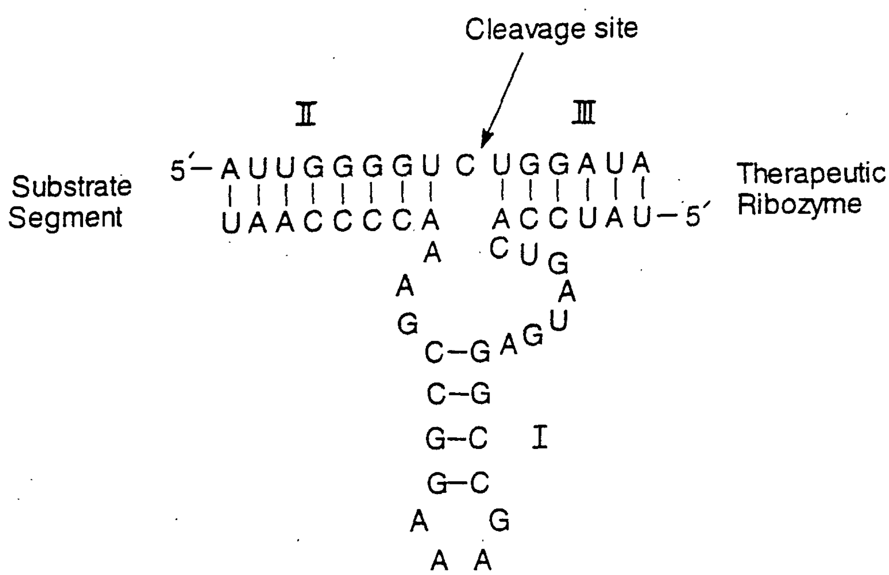


FIG. 1



Printed by Jouve, 75001 PARIS (FR)

**Description**

Background of the Invention

This invention relates to methods for inhibition of various animal diseases, including: a) inflammatory disease, in particular the inhibition of genetic expression which leads to the reduction or elimination of immune cell proliferation in inflammation or pre-inflammatory conditions; b) osteoarthritis, in particular, inhibition of genetic expression which leads to a reduction or elimination of extracellular matrix digestion. by matrix metalloproteinases; c) a stenotic condition, in particular the inhibition of genetic expression which. leads to the reduction or elimination of cellular proliferation in the areas of restenotic plaques; and d) cardiovascular disease such as hypertension, and in particular, those mediated by angiotensin converting enzyme activity.

Inflammatory disease

Inflammation is a tissue reaction to irritation, infection or injury marked by localized heat, swelling, redness, pain and sometimes loss of function. When tissues sustain physiological injury, the afflicted cells synthesize and/or release chemicals (cytokines and cellular adhesion molecules) which attract lymphoid cells and accelerate cell growth which is required for healing. The infiltration of lymphoid cells to the wound leads to the release of more biological response modifying molecules, which include vasoregulatory substances such as bradykinin and immunoregulatory molecules exemplified by the interleukin family of proteins. As healing occurs, the immune cell infiltration wanes and the inflammatory process ceases. Under certain physiological conditions, such as those observed in psoriasis, asthma, head injuries and systemic inflammatory response syndrome (SIRS), the condition persists because of inappropriate cellular controls. A persistent inflammatory condition may be acute or chronic and is generically referred to as inflammatory disease.

Inflammatory disease is exemplified by psoriasis, which is a skin disease caused by excessive growth of basal keratinocytes in the epidermis. It is believed that this uncontrolled growth of the keratinocytes results from stimulation by infiltrating T-cells. The conversion of normal skin to a psoriatic lesion is initiated by injury to the tissue and subsequent recognition of the injury by circulating immune cells. An acute inflammatory reaction ensues and results in the release of various cytokines and growth factors, including tumor necrosis. factor alpha (TNF-$\alpha$) and interleukin-1 (IL-1). A number of the cytokines released by infiltrating leukocytes induce production of cell adhesion molecules on the surface of the keratinocytes, while others induce the proliferation of basal keratinocytes and result in the release of keratinocyte factors which either auto-stimulate further growth or inhibit normal controls which limit keratinocyte proliferation. The injury further results in the local activation of T-cells which return to the circulation and may cause subsequent psoriatic conditions which are quite distant from the original site of injury.

Induction of the acute phase inflammatory reactants results in the production of IL-1 and TNF-alpha; both of these molecules induce the appearance of the T-cell homing proteins, I-CAM, ELAM-1 and VCAM-1 on the cell surface of keratinocytes. TNF-$\alpha$ also induces the production of TGF-$\alpha$, IL-6 and IL-8 by the keratinocytes. The cytokines and growth factors produced by the keratinocytes and infiltrating leukocytes cause uncontrolled keratinocyte proliferation. As the basal layer keratinocytes proliferate, the suprabasal keratinocytes are pushed closer to the surface of the skin where they normally form the cornified envelope layer and the stratum corneum. The abnormal rate of proliferation by the basal keratinocytes results in improper formation of the epidermis and stratum corneum and the ultimate development of the red, scaly appearance which is characteristic of psoriatic skin. The lack of keratinocyte growth regulation has been attributed to an unidentified mutation in a single gene or group of genes in which the keratinocytes no longer respond to the termination signal for wound healing.

There are 3 million patients in the United States afflicted with psoriasis. The available treatments for psoriasis are corticosteroids. The most widely prescribed are TEMOVATE (clobetasol propionate), LIDEX (fluocinonide), DIPROLENE (betamethasone propionate), PSORCON (diflorasone diacetate) and TRIAMCINOLONE formulated for topical application. The mechanism of action of corticosteroids is multifactorial and probably not due to simple inhibition of cell replication. This is a palliative therapy because the underlying cause of the disease remains, and upon discontinuation of the treatment the disease returns. Discontinuation of treatment is often prompted by the appearance of adverse effects such as atrophy, telangiectasias and purpura. Corticosteroids are not recommended for prolonged treatments or when treatment of large and/or inflamed areas is required. Alternative treatments include retinoids, such as etretinate, which has been approved for treatment of severe, refractory psoriasis. Alternative retinoid-based treatments are in advanced clinical trials. Retinoids act by converting keratinocytes to a differentiated state and restoration of normal skin development. Immunosuppressive drugs such as cyclosporine are also in the advanced stages of clinical trials. Due to the nonspecific mechanism of action of corticosteroids, retinoids and immunosuppressives, all current treatments of psoriasis exhibit severe side effects. and should not be used for extended periods of time unless the condition is life-threatening or disabling. There is a need for a less toxic, effective therapeutic agent in psoriatic patients.

Asthma affects nearly 5% of the population in industrialized nations, yet it is underdiagnosed and. undertreated. There is evidence that the incidence and prevalence of asthma are rising. These trends are occurring despite increases in the available therapies for asthma, which suggests that current methods of treating asthma are inadequate or not being utilized appropriately. Recently, it has been recognized that chronic asthma involves a characteristic inflammatory response in the airways.

Although it has long been acknowledged that fatal asthma is associated with inflammatory changes in the submucosal surfaces of the airways, it is now apparent that inflammation is present in patients with very mild asthma. Biopsies of patients have shown that infiltration of immune cells, especially eosinophils and lymphocytes, and epithelial shedding are prominent features. Further, there is a strong correlation between the degree of eosinophilia and the degree of bronchial hyperresponsiveness. Eosinophils are localized to areas of epithelial damage in the airways of patients. The basic proteins released by the eosinophils may be responsible for the damage observed in these patients. The role of mast cells and neutrophils in the disease is uncertain. Lymphocytes are present at the sites of tissue damage, but their role may be as mediators to amplify the eosinophilic response. In fact, interleukin-5, which is released by T-lymphocytes, is important in retaining and priming eosinophil action in the airway.

Arthritis

There are several types of arthritis, with osteoarthritis and rheumatoid arthritis being predominant. Osteoarthritis is a slowly progressive disease characterized by degeneration of articular cartilage with proliferation and remodeling of subchondral bone. It presents with a clinical picture of pain, deformity, and loss of joint motion. Rheumatoid arthritis is a chronic systemic inflammatory disease. Rheumatoid arthritis may be mild and relapsing or severe and progressive, leading to joint deformity and incapacitation.

Arthritis is the major contributor to functional impairment among the older population. It is the major cause of disability and accounts for a large proportion of the hospitalizations and health care expenditures of the elderly. Arthritis is estimated to be the principal cause of total incapacitation for about one million persons aged 55 and older and is thought to be an important contributing cause for about one million more.

Estimating the incidence of osteoarthritis is difficult for several reasons. First, osteoarthritis is diagnosed objectively on the basis of reading radiographs, but many people with radiologic evidence of disease have no obvious symptoms. Second, the estimates of prevalence are based upon clinical evaluations because radiographic data is not available for all afflicted joints. In the NHANESI survey of 1989, data were based upon a thorough musculoskeletal evaluation during which any abnormalities of the spine, knee, hips, and peripheral joints were noted as well as other specific diagnoses. Based on these observations, 12% of the US population between 25 and 74 years of age have osteoarthritis.

It is generally agreed that rheumatoid arthritis has a world-wide distribution and affects all racial and ethnic groups. The exact prevalence in the US is unknown but has been estimated to range between 0.5% and 1.5%. Rheumatoid arthritis occurs at all age levels and generally increases in prevalence with advancing age. It is 2-3 times more prevalent in women than in men and peak incidence occurs between 40-60 years of age. In addition to immunological factors, environmental, occupational and psychosocial factors have been studied for potential etiologic roles in the disease.

The extracellular matrix of multicellular organisms plays an important role in the formation and maintenance of tissues. The meshwork of the extracellular matrix is deposited by resident cells and provides a framework for cell adhesion and migration, as well as a permeability barrier in cell-cell communication. Connective tissue turnover during normal growth and development or under pathological conditions is thought to be mediated by a family of neutral metalloproteinases, which are zinc-containing enzymes that require calcium for full activity. The regulation of metalloproteinase expression is cell-type specific and may vary among species.

The best characterized of the matrix metalloproteinases, interstitial collagenase (MMP-1), is specific for collagen types I, II, and III. MMP-1 cleaves all three $\alpha$ chains of the triple helix at a single point initiating sequential breakdown of the interstitial. collagens. Interstitial collagenase activity has been observed in rheumatoid synovial cells as well as in the synovial fluid of patients with inflammatory arthritis. Gelatinases (MMP-2) represent a subgroup of the metalloproteinases consisting of two distinct gene products; a 70 kDa gelatinase expressed by most connective tissue cells, and a 92 kDa gelatinase expressed by inflammatory phagocytes and tumor cells. The larger enzyme is expressed by macrophages, SV-40 transformed fibroblasts, and neutrophils. The smaller enzyme is secreted by H-ras transformed bronchial epithelial cells and tumor cells, as well as normal human skin fibroblasts. These enzymes degrade gelatin (denatured collagen) as well as native collagen type XI. Stromelysin (MMP-3) has a wide spectrum of action on molecules composing the extracellular matrix. It digests proteoglycans, fibronectin, laminin, type IV and IX collagens and gelatin, and can remove the N-terminal propeptide region from procollagen, thus activating the collagenase. It has been found in human cartilage extracts, rheumatoid synovial cells, and in the synovium and chondrocytes of joints in rats with collagen-induced arthritis.

Both osteoarthritis and rheumatoid arthritis are treated mainly with compounds that inhibit cytokine or growth-factor induced synthesis of the matrix metalloproteinases which are involved in the extracellular matrix destruction observed in these diseases. Current clinical treatments rely upon dexamethasone and retinoid compounds, which are potent suppressors of a variety of metalloproteinases. The global effects of dexamethasone and retinoid treatment upon gene expression in treated cells make the development of alternative therapies desirable, especially for long term treatments. Recently, it was shown that gamma-interferon suppressed lipopolysaccharide induced collagenase and stromelysin production in cultured macrophages. Also, tissue growth factor-β (TGF-β) has been shown to block epidermal growth factor (EGF) induction of stromelysin synthesis in vitro. Experimental protocols involving gene therapy approaches include the controlled expression of the metalloproteinase inhibitors TIMP-1 and TIMP-2. Of the latter three approaches, only gamma-interferon treatment is currently feasible in a clinical application.

Stenosis

Stenosis is the occurrence of a blockage in a blood vessel. Such blockages may lead to impairment of functions or even death, dependent upon which vessel it occurs in and its size. These may be prevented as described below. One example of this condition is restenosis. Restenosis is a disease state which occurs as a sequelae to percutaneous transluminal angioplasty (PCTA) or coronary artery bypass grafting (CABG) treatments of cardiovascular disease. The condition is caused, primarily, by the proliferation of smooth muscle cells (atherophils) in the lamina propria layer of theintima in the vessel wall and secondarily, by the proliferation of other cell types present in the lamina propria. A secondary effect of the cellular proliferation is the increase in collagen and matrix protein synthesis. The cellular proliferation of the intimacytes and surrounding connective tissues results in intimal thickening, loss of vessel elasticity and reduction in blood flow through the afflicted region of the vessel.

While inflammation may play a role in the pathology of restenosis, other pathologic mechanisms are involved and possibly represent the underlying cause of the disease. Pathologic mechanisms associated with restenosis can be divided into three categories based upon when the restenosis occurs. The three categories and their mechanisms are 1) pre- and post-PCTA: thrombosis, platelet activation and thrombin generation; 2) immediate, after PCTA: elastic recoil; and 3) delayed; after PCTA: fibrocellular proliferation. Acute or immediate restenosis occurs in approximately 10% of the patients undergoing PCTA and slower-developing restenosis (six month onset) occurs in approximately 30% of those patients. The estimated number of PCTA patients in 1990 was 300,000 - 400,000. Thus, there are about 150,000 cases of restenosis per year in the United States.

The presently preferred chemotherapeutic treatment of patients is the use of streptokinase, urokinase or other thrombolytic compounds, such as fish oil, anticoagulants, ACE (angiotensin converting enzyme) inhibitors, aspirin and cholesterol lowering compounds; alternative treatment includes the surgical incorporation of endoluminal stents. It is reported that none of the current therapies have significantly impacted the rates of restenosis occurrence. A number of compounds are currently in preclinical evaluations. Platelet inhibitors include GR32191, Sultroban, Ketanserin, and fish oil. Angiopeptin is being tested as a growth factor inhibitor and Lovostatin, Enoxaparin, RD Heparin, Cilazapril and Fosinopril are being investigated as smooth muscle cell proliferation inhibitors. While platelet inhibitors are being tested for prevention of restenosis, it appears that these compounds will not be efficacious as short-term treatments. One of the biggest problems with current therapies is the occurrence of pharmacologic side-effects. These effects not only create other physiological problems, but also decrease the levels of patient compliance, thereby reducing the therapeutic efficacy of the treatments.

The proliferation of antherophils may be induced through a host of genetic activations, but the best candidate for targeting smooth muscle proliferation is the c-myb gene. The c-myb protein binds DNA and activates DNA replication and cellular growth. The role of c-myb in smooth muscle cell replication has been documented in bovine cells, and the expression of c-myb has been shown to activate cellular replication in chicken embryo fibroblasts and human T-lymphocytes.

Cellular growth factors may also play a role in local proliferation of intimacytes. Trauma to the area may induce the release of many factors such as TGF-β, PDGF, bFGF, endothelium-derived relaxing factor, CGRP and angiotensin II. Each of these factors could play a role in the induction of cellular proliferation in restenotic plaques, but these factors. are soluble proteins which exert their effects through secondary messenger systems in the target cells. One such messenger system is the NF-κB cascade. NF-κB protein activates cellular transcription and induces increases in cellular synthetic pathways. In a resting cell, this protein is found in the cytoplasm, complexed with its inhibitor, IκB. Upon phosphorylation of the IκB molecule, the complex dissociates and NFκB is released for transport to the nucleus, where it binds DNA and induces transcriptional activity in (NF-κB)-responsive genes. One of the (NF-κB)-responsive genes is the NF-κB gene. Thus, release of the NF-κB protein from the complex results in a cascade of gene expression which is auto-induced.

Summary of the Invention

The invention features use of ribozymes to treat or prevent various animal diseases, in particular, those human diseases noted above.

One such disease is psoriasis, which can be treated, e.g., by inhibiting the synthesis of tumor necrosis factor in activated lymphocytes, and basal keratinocytes. The invention also features use of ribozymes to treat chronic asthma, e.g., by inhibiting the synthesis of IL-5 in lymphocytes and preventing the recruitment and activation of eosinophils. Cleavage of targeted mRNAs (tumor necrosis factor and IL-5 mRNAs) expressed in keratinocytes, T-lymphocytes, monocytes or macrophages inhibits the synthesis of tumor necrosis factor and IL-5, respectively.

A number of other cytokines may also be involved in the activation of inflammation in asthmatic patients, including platelet activating factor, IL-1, IL-3, IL-4, GM-CSF, TNF$\alpha$, gamma interferon, ILAM-1, ELAM-1 and EoCSF. In addition to these cytokines, it is appreciated that any cellular receptors which mediate the activities of the cytokines are also good targets for intervention in inflammatory diseases. These targets include, but are not limited to, the IL-1R and TNF$\alpha$R on keratinocytes, epithelial and endothelial cells in airways. Recent data suggest that certain neuropeptides may play a role in asthmatic symptoms. These peptides include substance P, neurokinin A and calcitonin-gene-related peptides. These target genes may have more general roles in inflammatory diseases, but are currently assumed to have a role only in asthma. Other genes which are considered to play a role in asthma are the c-myb and c-myc genes, which may be triggered to induce endothelial cell proliferation and contribute to blockage of the airways. Those skilled in the art will recognize the other potential targets noted above are also suitable for treatment with ribozymes, which will reduce the risk or occurrence of inflammatory disease, such as the interleukins (1, 3, 4, 6, and 8), glycerol transferase, selectins (E-selectin, MEL-14), cell adhesion molecules (ICAM-1, ELAM-1, VCAM-1, GMP-140, MAM), TGF-$\alpha$, IL-1R, TGF$\alpha$R, EoCSF, $\alpha$-, $\beta$- or $\gamma$-interferon, EoCSF, GM-CSF and protein kinase C (PKC).

In particularly preferred embodiments, a ribozyme to TNF$\alpha$ nucleotides 374 to 393 can be use to inhibit TNF$\alpha$ protein production. This region may not a very good region to target because of secondary RNA structure in this region. An adjoining region which begins at nucleotide 380 in the previous reference and extends to nucleotide 412 (408-440, our sequence numbers see below) appears to contain a relatively more accessible RNA structure, and therefore represents an improved target over the region between nucleotides 374-393 (402-421, our sequence numbers).

Another disease is arthritis, which can be treated by inhibition of collagenase and stromelysin production in the synovial membrane of joints. Ribozyme treatment can be a partner to current treatments which primarily target immune cells reacting to pre-existing tissue damage. Early ribozyme treatment which reduces the collagenase or stromelysin-induced damage can be followed by treatment with the anti-inflammatories or retinoids, if necessary. In this manner, expression of the proteinases can be controlled at both transcriptional and translational levels. Ribozyme treatment can be given to patients expressing radiological signs of osteoarthritis prior to the expression of clinical symptoms. Ribozyme treatment can impact the expression of stromelysin without introducing the non-specific effects upon gene expression which accompany treatment with the retinoids and dexamethasone. The ability of stromelysin to activate procollagenase indicates that a ribozyme which reduces stromelysin expression can also be used in the treatment of both osteoarthritis (which is primarily a stromelysin-associated pathology) and rheumatoid arthritis (which is primarily related to enhanced collagenase activity).

While a number of cytokines and growth factors induce metalloproteinase activities during wound healing and tissue injury of a pre-osteoarthritic condition, these molecules are not preferred targets for therapeutic intervention. Primary emphasis is placed upon inhibiting the molecules which are responsible for the disruption of the extracellular matrix, because most people will be presenting radiologic or clinical symptoms prior to treatment. The most versatile of the metalloproteinases (the molecule which can do the most structural damage to the extracellular matrix, if not regulated) is stromelysin. Additionally, this molecule can activate procollagenase, which in turn causes further damage to the collagen backbone of the extracellular matrix. Under normal conditions, the conversion of prostromelysin to active stromelysin is regulated, by the presence of inhibitors called TIMPs (tissue inhibitors of MMP). Because the level of TIMP in synovial cells exceeds the level of prostromelysin and stromelysin activity is generally absent from the synovial fluid associated with non-arthritic tissues, the toxic effects of inhibiting stromelysin activity in non-target cells should be negligible.

Thus, the invention features use of ribozymes to treat or prevent arthritis, particularly osteoarthritis, e.g., by inhibiting the synthesis of the prostromelysin molecule in synovial cells, or by inhibition of other matrix metalloproteinases discussed above. Cleavage of targeted mRNAs (stromelysin mRNAs) expressed in macrophages, neutrophils and synovial cells represses the synthesis of the zymogen form of stromelysin, prostromelysin. Those in the art will recognize the other potential targets discussed above are also suitable for treatment with ribozymes, which will reduce the risk or occurrence of pathologic degradation of the extracellular matrix such as the collagenase and gelatinase metalloproteinases, other proteinases which can activate the proenzyme forms of the metalloproteinases in synovial fluid or cartilaginous cells, cytokines or growth factors which activate the expression of the metalloproteinases and adhesion molecules which attract macrophage and neutrophils to the areas of tissue injury.

Another disease is stenosis, which is treated, e.g., by inhibiting the activation of smooth muscle proliferation by inhibiting the expression of the cellular c-myb gene. Cleavage of targeted mRNAs (c-myb mRNAs) expressed in endothelial cells and smooth muscle cells represses activation of cellular replication and abnormal proliferation of the smooth muscle or endothelial cells. Other potential targets suitable for treatment with ribozymes, which will reduce the risk or occurrence of cellular proliferation in the areas of restenotic risk include mRNAs encoding TGF-β, NF-κB, PDGF, bFGF, endothelium-derived relaxing factor, CGRP, and angiotensin II.

Yet another disease is cardiovascular disease, which can be treated, e.g., by inhibiting the activation of angiotensin by angiotensin converting enzyme (ACE), or by inhibition of the activity of endothelin converting enzyme (ECE). Cleavage of targeted mRNAs (ACE mRNAs) expressed in endothelial cells elicits decreased levels of the vasoactive form of angiotensin. Those in the art will recognize many other potential targets suitable for treatment with ribozymes, which will reduce the risk or occurrence of cardiovascular disease, such as mRNAs encoding HMG CoA reductase, renin, bradykinin, plasminogen, factors IX, X and II, 2-5A synthetase, ADH and fibrinogen.

Ribozymes are RNA molecules having an enzymatic activity which is able to repeatedly cleave other separate RNA molecules in a nucleotide base sequence specific manner. It is alleged that such enzymatic RNA molecules can be targeted to virtually any RNA transcript and efficient cleavage has been achieved *in vitro.* Kim et al., 84 Proc. Natl. Acad. Sci. USA 8788, 1987; Haseloff and Gerlach, 334 Nature 585, 1988; Cech, 260 JAMA 3030, 1988; and Jefferies et al., 17 Nucleic Acids Research 1371, 1989.

Ribozymes act by first binding to a target RNA. Such binding occurs through the target RNA binding portion of a ribozyme which is held in close proximity to an enzymatic portion of the RNA which acts to cleave the target RNA. Thus, the ribozyme first recognizes and then binds a target RNA through complementary base-pairing, and once bound to the correct site, acts enzymatically to cut the target RNA. Strategic cleavage of such a target RNA will destroy its ability to direct synthesis of an encoded protein. After a ribozyme has bound and cleaved its RNA target it is released from that RNA to search for another target and can repeatedly bind and cleave new targets.

The enzymatic nature of a ribozyme is advantageous over other technologies, such as antisense technology (where a nucleic acid molecule simply binds to a nucleic acid target to block its translation) since the effective concentration of ribozyme necessary to effect a therapeutic treatment is lower than that of an antisense oligonucleotide. This advantage reflects the ability of the ribozyme to act enzymatically. Thus, a single ribozyme molecule is able to cleave many molecules of target RNA. In addition, the ribozyme is a highly specific inhibitor, with the specificity of inhibition depending not only on the base pairing mechanism of binding, but also on the mechanism by which the molecule inhibits the expression of the RNA to which it binds. That is, the inhibition is caused by cleavage of the RNA target and so specificity is defined as the ratio of the rate of cleavage of the targeted RNA over the rate of cleavage of non-targeted RNA. This cleavage mechanism is dependent upon factors additional to those involved in base pairing. Thus, it is thought that the specificity of action of a ribozyme is greater than that of antisense oligonucleotide binding the same RNA site.

This class of chemicals exhibits a high degree of specificity for cleavage of the intended target mRNA. Consequently, the ribozyme agent will only affect cells expressing that particular gene, and will not be toxic to normal tissues.

The invention can be used to treat or prevent (prophylactically) psoriasis, asthma and other inflammatory diseases, and restenosis or other cardiovascular conditions, including hypertension. The preferred administration protocol is *in vivo* administration to reduce the level of those genes and the encoded mRNAs noted above.

Thus, in the first aspect, the invention features an enzymatic RNA molecule (or ribozyme) which cleaves mRNA associated with development or maintenance of a psoriatic or asthmatic condition, e.g., mRNA encoding TNF-α, IL-5, IL-1, IL-3, IL-4, IL-6, IL-8, glycerol transferase, selectins, E-selectin, MEL-14, ICAM-1, ELAM-1, VCAM-1, GMP-140, MAM, TGFα, TNFαR, IL-1R, α-, β- or γ-interferon, GM-CSF and protein kinase C, and in particular, those mRNA targets disclosed in Table 1.

Table 1

| TNF-α mRNA Nucleotide Number | Sequence | SEQ.ID.NO. |
| --- | --- | --- |
| 27 | CAGCAGAGGACCAGCUA | ID.NO. 01 |
| 56 | GCAACUACAGACCC | ID.NO. 02 |
| 70 | CCCCUGAAAACAACCCUCAGACGC | ID.NO. 03 |
| 94 | CACAUCCCCUGACAAGCUGCCAGGCAGG | ID.NO. 04 |
| 134 | CACAUACUGACCCA | ID.NO. 05 |
| 157 | CCUCUCUCCCCUGGAAAGG | ID.NO. 06 |

| | | |
|---|---|---|
| 176 | ACACCAUGAGCACUGAAAGCAUGAUCCGGGAC | ID.NO. 07 |
| 253 | GCCCCAGGGCUCCAGGC | ID.NO. 08 |
| 270 | GGUGCUUGUUCCUCAGCCUCUUC | ID.NO. 09 |
| 311 | CCACCACGCUCUUC | ID.NO. 10 |
| 362 | AAGAGUUCCCCAG | ID.NO. 11 |
| 408 | AGUCAGAUCAUCUUCUCGAACCCCGAGUGACAA | ID.NO. 12 |
| 446 | UAGCCCAUGUUGUAGCAAACCCUCAAGCUGAGGG | ID.NO. 13 |
| 574 | UACCUCAUCUACUCC | ID.NO. 14 |
| 599 | UCAAGGGCCAAGGCUGCCCCUC | ID.NO. 15 |
| 621 | CACCCAUGUGCUCCUCACCCA | ID.NO. 16 |
| 652 | CGCAUCGCCGUCUCCUACCAGACCAA | ID.NO. 17 |
| 694 | GCCAUCAAGAG | ID.NO. 18 |
| 824 | CCGACUAUCUCGACUUUGCC | ID.NO. 19 |
| 869 | UCAUUGCCCUGUGAGGAGGACGAACAUCCAACCU | ID.NO. 20 |
| 991 | CUUAGGGUCGGAACCCAA | ID.NO. 21 |
| 1043 | AAACCUGGGAUUCAGGAA | ID.NO. 22 |
| 1084 | CUGGCAACCACUAAGAAU | ID.NO. 23 |
| 1115 | UCCAGAACUCACUGG | ID.NO. 24 |
| 1187 | UGGCCAGAAUGCUGCAGGACUUGAGA | ID.NO. 25 |
| 1213 | AGACCUCACCUAGAAAUUGACACAAGU | ID.NO. 26 |
| 1276 | CUUCCUUGAGA | ID.NO. 27 |
| 1322 | UCUAUUUAUGUUUGCACUUGUG | ID.NO. 28 |
| 1344 | AUUAUUUAUUAUUUAUUUAUUAUUUAUUUAUUUA | ID.NO. 29 |
| 1378 | CAGAUGAAUGUAUUUAUUU | ID.NO. 30 |
| 1402 | ACCGGGGUAUCC | ID.NO. 31 |
| 1419 | GACCCAAUGUAGGAGCUGCCUUGGCUCAG | ID.NO. 32 |
| 1495 | AGCCCCCUGGC | ID.NO. 33 |
| 1513 | CCUUCUUUUGAUUAUGUUUUUUAAAAUAUUUAUC | ID.NO. 34 |
| 1557 | GUCUAAACAAUGCU | ID.NO. 35 |
| 1587 | GUCACUCAUUGCUGAG | ID.NO. 36 |
| 1639 | CUACUAUUCAGU | ID.NO. 37 |
| 1656 | GAAAUAAAGUUUGCUU | ID.NO. 38 |

| IL-5 mRNA Nucleotide Number | Sequence | SEQ.ID.NO. |
|---|---|---|
| 10 | CUUUGCCAAAGGCAAAC | ID.NO. 39 |

| | | |
|---|---|---|
| 33 | CGUUUCAGAGCCAUGAGGAUGC | ID.NO. 40 |
| 61 | AUUUGAGUUUGCUAGCUCUUGGAGCUG | ID.NO. 41 |
| 88 | CCUACGUGUAUGCCAUCC | ID.NO. 42 |
| 139 | AGACCUUGGCACUG | ID.NO. 43 |
| 158 | UACUCAUCGAACUCUGCUGAUA | ID.NO. 44 |
| 209 | UGUACAUAAAAA | ID.NO. 45 |
| 275 | AAACUGUGCAA | ID.NO. 46 |
| 299 | AAAGACUAUUCAAAAACUUGUCC | ID.NO. 47 |
| 370 | AGACGGAGAGUAAACCAAUUCCUAGACUACCUGC | ID.NO. 48 |
| 522 | AAGAAAGAGUCA | ID.NO. 49 |
| 558 | ACUUCAGAGGGAAAG | ID.NO. 50 |
| 578 | AUUUCAGGCAUACUGACACUUUGCCAGAAAGCA | ID.NO. 51 |
| 635 | AUAUCAGAAUCA | ID.NO. 52 |
| 667 | CAAAAUUGAUAUACUUUUUUCUUAUUUAA | ID.NO. 53 |
| 738 | GAAAUGGUUAAGAAUUUG | ID.NO. 54 |

In a second aspect, the invention features and enzymatic RNA molecule which cleaves mRNA associated with development and maintenance of osteoarthritis or other pathological conditions which are mediated by metalloproteinase activation. The preferred administration protocol is *in vivo* administration to reduce the level of stromelysin activity.

Thus, in this aspect, the invention features an enzymatic RNA molecule (or ribozyme) which cleaves mRNA associated with development or maintenance of an arthritic condition, e.g., mRNA encoding stromelysin, and in particular, those mRNA targets disclosed in Table 2.

Table 2

| Nucleotide Number | Sequence | SEQ. ID. NO. |
|---|---|---|
| 20 | UAGAGCUAAGUAAAGCCAG | ID.NO. 01 |
| 126 | ACACCAGCAUGAA | ID.NO. 02 |
| 147 | AGAAAUAUCUAGA | ID.NO. 03 |
| 171 | ACCUCAAAAAAGAUGUGAAACAGU | ID.NO. 04 |

| | | |
|---|---|---|
| 240 | AAAUGCAGAAGUUC | ID.NO. 05 |
| 287 | GACACUCUGGAGGUGAUGCGCAAGCCCAGGUGU | ID.NO. 06 |
| 327 | CUGAUGUUGGUCACUUCAGAAC | ID.NO. 07 |
| 357 | GCAUCCCGAAGUGGAGGAAAACCCACCUUACAU | ID.NO. 08 |
| 402 | AUUAUACACCAGAUUUGCCAAAAGAUG | ID.NO. 09 |
| 429 | CUGUUGAUUCUGCUGUUGAGA | ID.NO. 10 |
| 455 | CUGAAAGUCUGGGAAGAGGUGA | ID.NO. 11 |
| 513 | CUGAUAUAAUGA | ID.NO. 12 |
| 592 | UGCCUAUGCCCC | ID.NO. 13 |
| 624 | AUGCCCACUUUGAUGAUGAUGAACAAUGGACA | ID.NO. 14 |
| 679 | AUUUCUCGUUGCUGCUCAUG | ID.NO. 15 |
| 725 | CACUCAGCCAACACUGA | ID.NO. 16 |
| 801 | AAGAUGAUAUAAAUGGCAUUCAGUCC | ID.NO. 17 |
| 827 | CUCUAUGGACCUCCCCCUGACUCCCCU | ID.NO. 18 |
| 859 | CCCCCUGGUACCCA | ID.NO. 19 |
| 916 | UCCUGCUUUGUCCUUUGAUGCUGUCAGCAC | ID.NO. 20 |
| 958 | AAUCCUGAUCUUUAAAGA | ID.NO. 21 |
| 975 | CAGGCACUUUUGGCGCAAAUCCC | ID.NO. 22 |
| 1018 | AUUGCAUUUGAUCUCUUCAUUUUGGCCAUC | ID.NO. 23 |
| 1070 | GCAUAUGAAGUUA | ID.NO. 24 |
| 1203 | AAAUCGAUGCAGCCAUUUCUGA | ID.NO. 25 |
| 1274 | UUUGAUGAGAAGAGAAAUUCCAUGGAGC | ID.NO. 26 |
| 1302 | CAGGCUUUCCCAAGCAAAUAGCUGAAGAC | ID.NO. 27 |
| 1420 | CCCAAAUGCAAAG | ID.NO. 28 |
| 1485 | AUGUAGAAGGCACAAUAUGGGCACUUUAAA | ID.NO. 29 |
| 1623 | UCUUGCCGGUCAUUUUUAUGUUAU | ID.NO. 30 |
| 1665 | GCUGCUGCUUAGC | ID.NO. 31 |
| 1733 | CAACAGACAAGUGACUGUAUCU | ID.NO. 32 |
| 1769 | CUUAUUUAAUA | ID.NO. 33 |

In the third aspect, the invention features an enzymatic RNA molecule (or ribozyme) which cleaves mRNA associated with development or maintenance of a restenotic condition, e.g., mRNA encoding c-myb (or other mRNAs noted above), and in particular, those mRNA targets disclosed in Table 3.

Table 3

| Nucleotide Number | Sequence | Seq. ID. No. |
|---|---|---|
| 1 | GGCGGCAGCGCCCUGCCGACGCCGGGG | ID.NO.01 |
| 77 | CCGCGGCUCUCGGC | ID.NO.02 |
| 111 | GCCAUGGCCCGAA | ID.NO.03 |
| 129 | CGGCACAGCAUAUAUAGCAGUGACGAGGA | ID.NO.04 |
| 165 | GACUUUGAGAUGUGUGACCAUGACUAUGAUGGG | ID.NO.05 |
| 211 | CUGGAAAGCGUC | ID.NO.06 |
| 248 | GGAAGAGGAUGAAAAACUGAAGAAG | ID.NO.07 |
| 267 | GAAGAACUGGUGGAACAGAAUGGAAC | ID.NO.08 |
| 299 | CUGGAAAGUUAUUGCCAA | ID.NO.09 |
| 323 | CCCGAAUCGAACAGAUGUGCAG | ID.NO.10 |
| 362 | GAAAGUACUAAACCCUGAG | ID.NO.11 |
| 394 | CUUGGACCAAAGAAGAAGAUCAGAGAGUGAUA | ID.NO.12 |
| 433 | ACAGAAAUACGGUCCGAAACGUUGGUCUG | ID.NO.13 |
| 463 | UUAUUGCCAAGCACUUAAAGGGGAGAAUUGGAA | ID.NO.14 |
| 527 | GAAUCCAGAAGUUAAGAA | ID.NO.15 |
| 563 | GGAAGACAGAAUUAUUUACCAGGCACA | ID.NO.16 |
| 590 | CAAGAGACUGGGGAACAGAU | ID.NO.17 |
| 616 | AAAUCGCAAAGCUA | ID.NO.18 |
| 636 | GGACGAACUGAUAAUGCUAUCAAGAACC | ID.NO.19 |
| 664 | ACUGGAAUUCUACAAUGCGUCGGAAGGUCGAACA | ID.NO.20 |
| 732 | CAGCCAGCAGUGGCCACAA | ID.NO.21 |
| 768 | CAUUUGAUGGGUUUUGCUCAGGCUCCGCCUACA | ID.NO.22 |
| 801 | GCUCAACUCCCUGCCACUGGCCAGCCC | ID.NO.23 |
| 834 | AACAACGACUAUUCCUAUUACCACA | ID.NO.24 |
| 870 | CAAAAUGUCUCCAGUCAUGUUCCAUACCCU | ID.NO.25 |
| 914 | AAAUAUAGUCAAUGUCCCUCAGCCAGCUGCCGCA | ID.NO.26 |
| 955 | AGAGACACUAUAAUGAUGAAGACCCUGAGAAGGA | ID.NO.27 |
| 989 | AAAGCGAAUAAAGGAAUUAGAAUUG | ID.NO.28 |
| 3179 | CGGUGUACUUACUGCC | ID.NO.29 |
| 1036 | AGCUAAAAGGACAGCAGGUGCUACCAACACAGAA | ID.NO.30 |
| 1086 | CCCGGGUGGCACAGCACCACCAUUGCCGACCACA | ID.NO.31 |
| 1162 | AACACCACUCCACUCCAUCUCUGCCAGCGGAUCC | ID.NO.32 |
| 1204 | UACCUGAAGAAA | ID.NO.33 |

| | | |
|---|---|---|
| 1236 | AUGAUCGUCCACCAGGGCACCAUU | ID.NO.34 |
| 1291 | CAGAAACACUCCAAUUUA | ID.NO.35 |
| 1343 | AAACUCAGACU | ID.NO.36 |
| 1359 | AUGCCUUCUUUAAC | ID.NO.37 |
| 1405 | UUACAACACCA | ID.NO.38 |
| 1440 | ACUCAAAAGGAAAAUACUGUUUUUAGAACCC | ID.NO.39 |
| 1471 | CAGCUAUCAAAAGGUCAAUCUUAGAAAGCU | ID.NO.40 |
| 1501 | CUCCAAGAACUCCUACACCAUUCAA | ID.NO.41 |
| 1526 | ACAUGCACUUGCAGCUCAAGAA | ID.NO.42 |
| 1554 | UACGGUCCCCUGAAGAUGCUACCUCAGA | ID.NO.43 |
| 1582 | CACCCUCUCAUCUAGUAGAAGAUCUGCAGGA | ID.NO.44 |
| 1618 | UCAAACAGGAAUCUGAUGAAUCUGGA | ID.NO.45 |
| 1660 | AAGAAAAUGGA | ID.NO.46 |
| 1676 | CUUACUGAAGAAAAUCAAACAAGA | ID.NO.47 |
| 1705 | AAUCUCCAACUGAUAAAUCAG | ID.NO.48 |
| 1738 | GCUCACACCACUGGGA | ID.NO.49 |
| 1789 | CCUCGCCUGUGCGAGAUGCACCGAAUAUUC | ID.NO.50 |
| 1838 | GGCACCAGCAUCAGAAGAUGAAGAC | ID.NO.51 |
| 1876 | CAUUUACAGUACC | ID.NO.52 |
| 1900 | CCCUGGCGAGCCCCUUGCA | ID.NO.53 |
| 1919 | GCCUUGUAGCAGUACCUGGGA | ID.NO.54 |
| 1984 | GUCAAGCUCGUAAAUACGUGAA | ID.NO.55 |
| 2067 | GAACAGUUCAA | ID.NO.56 |
| 2106 | AUGAAACUUUUCAU | ID.NO.57 |
| 2229 | AAAAUAAAUAACAGUC | ID.NO.58 |
| 2265 | UGAAUUGUAGCC | ID.NO.59 |
| 2282 | UUAAUAUCUUAAU | ID.NO.60 |
| 2325 | AUUUAUCUGGUAUUUUAAAGGAUCCAACAGAUC | ID.NO.61 |
| 2410 | CCAGUAUUUCA | ID.NO.62 |
| 2426 | CUCGAUCACUAAACAUAUG | ID.NO.63 |
| 2445 | CAUAUAUUUUAAAAAUC | ID.NO.64 |
| 2695 | UGCUAUGGUCUUAGCCU | ID.NO.65 |
| 2726 | AGUAUCAGAGG | ID.NO.66 |
| 2776 | UAGGUAAUUGACUAU | ID.NO.67 |
| 2798 | UAUUUCAGACUUUUUAAUUUUAUAUAUAUAUACA | ID.NO.68 |
| 2847 | CAAUACAUUUGAAAACUUGUUUGGGAGACUCUGC | ID.NO.69 |
| 2891 | GUGGUUUUUUUGUUAUUGUUGGUUU | ID.NO.70 |

| | | |
|---|---|---|
| 2935 | UUCUUUUUUGGGAGAU | ID.NO.71 |
| 2967 | CUAUGUUUUGUUUUG | ID.NO.72 |
| 2987 | AGCCUGACUGUUUUAUA | ID.NO.73 |
| 3016 | UCGAUUUGAUC | ID.NO.74 |
| 3072 | UGGAUCCUGUGUU | ID.NO.75 |
| 3111 | UUGAUAGCCAGUCACUGCCUUAAGA | ID.NO.76 |
| 3136 | ACAUUUGAUGCAAGAUGGCCAGCACU | ID.NO.77 |

In a fourth aspect, the invention features an enzymatic RNA molecule (or ribozyme) which cleaves mRNA associated with development or maintenance of a cardiovascular condition, e.g., mRNA encoding ACE or ECE, and in particular, those mRNA targets disclosed in Table 4.

Table 4

| Nucleotide Number | Sequence | Seq. ID. No. |
|---|---|---|
| 38 | GCUACUGCAGGACUUCCCAGC | ID.NO. 01 |
| 59 | CUCCUCUUCCUGCUGCUCGCUAGG | ID.NO. 02 |
| 105 | GCCAGGAGGCAUC | ID.NO. 03 |
| 120 | AACAGGUGACAGUCACCCAUG | ID.NO. 04 |
| 210 | CCCAGAGCCCAAACCUGGUGA | ID.NO. 05 |
| 247 | CAGCAAGUUUGUGGAGGAAUAUGA | ID.NO. 06 |
| 271 | CCGGACAUCCCA | ID.NO. 07 |
| 292 | GAACGAGUAUGCCGAGGCC | ID.NO. 08 |
| 311 | AACUGGAACUACAACAC | ID.NO. 09 |
| 341 | GAGACCAGCAAGAUUCUGCUG | ID.NO. 10 |
| 369 | ACAUGCAAAUAG | ID.NO. 11 |
| 387 | ACACCCUGAAGUACGGCACCCAG | ID.NO. 12 |
| 424 | GUGAACCAGUUGCAGAACACCACUA | ID.NO. 13 |
| 474 | AGGACCUAGAA | ID.NO. 14 |
| 491 | GCGCUGCCUGCCCAGGAGCUGGAG | ID.NO. 15 |
| 515 | GAGUACAACAAGAUCCUGUUGGA | ID.NO. 16 |
| 535 | GGAUAUGGAAACCACCUACAGC | ID.NO. 17 |
| 564 | CUGUGUGCCACCCGAAUGGC | ID.NO. 18 |
| 598 | CGAGCCAGAUCUGACGAAUGUGAUG | ID.NO. 19 |

| | | |
|---|---|---|
| 627 | CAUCCCGGAAAUAUGAAGACCUG | ID.NO. 20 |
| 646 | CCUGUUAUGGGCAUGGG | ID.NO. 21 |
| 667 | CUGGCGAGACAAGGCGGG | ID.NO. 22 |
| 706 | CCCGAAAUACG | ID.NO. 23 |
| 725 | AUCAACCAGG | ID.NO. 24 |
| 755 | GUAGAUGCAGGGGACUC | ID.NO. 25 |
| 775 | AGGUCUAUGUACGAGACACCAUCC | ID.NO. 26 |
| 831 | AGCUGCAGCCACUCUACCUCAAC | ID.NO. 27 |
| 844 | CUGCAUGCCUACGUGCGCCG | ID.NO. 28 |
| 899 | CAGCAUCAAC | ID.NO. 29 |
| 921 | CCAUUCCUGCUCAC | ID.NO. 30 |
| 956 | CAGACCUGGUCCAAC | ID.NO. 31 |
| 971 | AUCUAUGACUUGGUGG | ID.NO. 32 |
| 996 | CUUCAGCCCCCUCGAUGGAC | ID.NO. 33 |
| 1015 | CACCACAGAGGCUAUGCUAA | ID.NO. 34 |
| 1040 | GGCUGGACGC | ID.NO. 35 |
| 1054 | GAGGAUGUUUAAGGAGGCUGAUGA | ID.NO. 36 |
| 1071 | CUGAUGAUUUCUUCACCUCC | ID.NO. 37 |
| 1107 | UGCCUCCUGAGUUCUGGAACA | ID.NO. 38 |
| 1127 | AAGUCGAUGCUGGAGAAG | ID.NO. 39 |
| 1173 | ACGCCUCGGCCUGGGACUUCUACAA | ID.NO. 40 |
| 1203 | AGGACUUCCGGAUCAAGCAGUGCA | ID.NO. 41 |
| 1227 | CCACCGUGAACUUGGAGGACCUGG | ID.NO. 42 |
| 1275 | ACAUCCAGUAUUUC | ID.NO. 43 |
| 1291 | GCAGUACAAAGACUUACCUGUGG | ID.NO. 44 |
| 1315 | CUUGAGGGAGGGUGCCAACC | ID.NO. 45 |
| 1335 | CCGGCUUCCAUGAGGCCAUUGG | ID.NO. 46 |
| 1358 | GACGUGCUAG | ID.NO. 47 |
| 1376 | GUGUCUACGCCCAAGCACCUGCACA | ID.NO. 48 |
| 1401 | GUCUCAACCUGCUGAGCAG | ID.NO. 49 |
| 1429 | CAGCGACGAGCAUGACAUCAAC | ID.NO. 50 |
| 1450 | CUUUCUGAUGAAGAUGGCCCUUG | ID.NO. 51 |
| 1469 | CUUGACAAGAUCGCC | ID.NO. 52 |
| 1500 | ACCUCGUCGAUCAGUGGCG | ID.NO. 53 |
| 1536 | GAAGCAUCACC | ID.NO. 54 |
| 1553 | AACUAUAACCAGGAGUGG | ID.NO. 55 |
| 1565 | GCCUCAGGCUGAAGUA | ID.NO. 56 |

| | | |
|---|---|---|
| 1591 | CCAGGGCCUCUGCCCCCCAG | ID.NO. 57 |
| 1616 | AGGACUCAAGGUGAC | ID.NO. 58 |
| 1630 | CUUUGACCCAGGGGCC | ID.NO. 59 |
| 1662 | CUAGCGUGCCUUAC | ID.NO. 60 |
| 1699 | CAUCCAGUUCCAGUUCCACG | ID.NO. 61 |
| 1701 | GUUCCACGAGGCACUG | ID.NO. 62 |
| 1749 | GCCCCCUGCACAAGUGUGACAUC | ID.NO. 63 |
| 1771 | CUACCAGUCCAAGGAG | ID.NO. 64 |
| 1894 | GAGCUACUUCAAGCUGCUGG | ID.NO. 65 |
| 1915 | GGACUGGCUCCGCACGG | ID.NO. 66 |
| 1968 | AGUACAACUGGACGCC | ID.NO. 67 |
| 1984 | GAACUCCGCUCGCUCAGAAGG | ID.NO. 68 |
| 2005 | GCCCCUCCCAGACAG | ID.NO. 69 |
| 2046 | ACCUGGAUGCGCAGCA | ID.NO. 70 |
| 2076 | AGUGGCUGCUGC | ID.NO. 71 |
| 2101 | CGCCCUGCUGGUAGCCACCC | ID.NO. 72 |
| 2147 | AUCCGCCACCGCAGCCUCC | ID.NO. 73 |
| 2212 | ACACUCCUGAGGUGACCCGG | ID.NO. 74 |
| 2316 | GCCCACCCUGC | ID.NO. 75 |
| 2337 | CUGUCCCUGUCCCCCUCCCC | ID.NO. 76 |
| 2365 | CUCCAGACCACC | ID.NO. 77 |
| 2386 | AGCCCCUUCUCCCAGCACAC | ID.NO. 78 |
| 2408 | CUGCCUGACACUGAGCCC | ID.NO. 79 |
| 2426 | CACCUCUCCAAGUCUCUCUG | ID.NO. 80 |
| 2446 | UGAAUACAAUUAAAGGUCCUG | ID.NO. 81 |

By "enzymatic RNA molecule" it is meant an RNA molecule which has complementarity in a substrate binding region to a specified mRNA target, and also has an enzymatic activity which is active to specifically cleave that mRNA. That is, the enzymatic RNA molecule is able to intermolecularly cleave mRNA and thereby inactivate a target mRNA molecule. This complementarity functions to allow sufficient hybridization of the enzymatic RNA molecule to the target RNA to allow the cleavage to occur. One hundred percent complementarity is preferred, but complementarity as low as 50-75% may also be useful in this invention. For *in vivo* treatment, complementarity between 30 and 45 bases is preferred.

In preferred embodiments, the enzymatic RNA molecule is formed in a hammerhead motif, but may also be formed in the motif of a hairpin, hepatitis delta virus, group I intron or RNaseP-like RNA (in association with an RNA guide sequence). Examples of such hammerhead motifs are described by Rossi et al., 8 Aids Research and Human Retroviruses 183, 1992, of hairpin motifs by Hampel and Tritz, 28 Biochemistry 4929, 1989; and Hampel et al., 18 Nucleic Acids Research 299, 1990, and an example of the hepatitis delta virus motif is described by Perrotta and Been, 31 Biochemistry 16, 1992, of the RNaseP motif by Guerrier-Takada et al., 35 Cell 849, 1983, and of the group I intron by Cech et al., U.S. Patent 4,987,071. These specific motifs are not limiting in the invention and those skilled in the art will recognize that all that is important in an enzymatic RNA molecule of this invention is that it has a specific substrate binding site which is complementary to one or more of the target gene RNA regions, and that it have nucleotide sequences within or surrounding that substrate binding site which impart an RNA cleaving activity to the molecule.

In a related aspect, the invention features a mammalian cell which includes an enzymatic RNA molecule as described above. Preferably, the mammalian cell is a human cell.

In another related aspect, the invention features an expression vector which includes nucleic acid encoding an enzymatic RNA molecule described above, located in the vector, e.g., in a manner which allows expression of that enzymatic RNA molecule within a mammalian cell.

In yet another related aspect, the invention features a method for treatment of a disease noted above by administering to a patient an enzymatic RNA molecule as described above.

The invention provides a class of chemical, cleaving agents which exhibit a high degree of specificity for the mRNA causative of a psoriatic or arthritic or cardiovascular condition. Such a condition includes any measurable indication of susceptibility to cardiac problems, and thus includes predisposition to such conditions or cardiovascular disease. Such enzymatic RNA molecules can be delivered exogenously or endogenously to infected cells. In the preferred hammerhead motif the small size (less than 40 nucleotides, preferably between 32 and 36 nucleotides in length) of the molecule allows the cost of treatment to be reduced.

The smallest ribozyme delivered for any type of treatment reported to date (by Rossi et al., 1992, *supra*) is an *in vitro* transcript having a length of 142 nucleotides. Synthesis of ribozymes greater than 100 nucleotides in length is very difficult using automated methods, and the therapeutic cost of such molecules is prohibitive. Delivery of ribozymes by expression vectors is primarily feasible using only *ex vivo* treatments. This limits the utility of this approach. In this invention, an alternative approach uses smaller ribozyme motifs (e.g., of the hammerhead structure, shown generally in Fig. 1), and exogenous delivery.. The simple structure of these molecules also increases the ability of the ribozyme to invade targeted regions of the mRNA structure. Thus, unlike the situation when the hammerhead structure is included within longer transcripts, there are no non-ribozyme flanking sequences to interfere with correct folding of the ribozyme structure, as well as complementary binding of the ribozyme to the mRNA target.

The enzymatic RNA molecules of this invention can be used to treat psoriatic or pre-psoriatic, asthmatic or pre-asthmatic, arthritic or prearthritic, stenotic or prestenotic conditions. Such treatment can also be extended to other related genes in nonhuman primates. Affected animals can be treated at the time of disease risk or detection, or in a prophylactic manner. This timing of treatment will reduce the chance of further disease damage.

Ribozymes of this invention may be used as diagnostic tools to examine genetic drift and mutations within diseased cells. The close relationship between ribozyme activity and the structure of the target RNA allows the detection of mutations in any region of the molecule which alters the base-pairing and three-dimensional structure of the target RNA. By using multiple ribozymes described in this invention, one may map nucleotide changes which are important to RNA structureand function *in vitro,* as well as in cells and tissues. Cleavage of target RNAs with ribozymes may be used to inhibit gene expression and define the role (essentially) of specified gene products in the progression of disease. In this manner, other genetic targets may be defined as important mediators of the disease. These experiments will lead to better treatment of the disease progression by affording the possibility of combinational therapies (e.g., multiple ribozymes targeted to different genes., ribozymes coupled with known small molecule inhibitors, or intermittent treatment with combinations of ribozymes and/or other chemical or biological molecules).

Other features and advantages of the invention will be apparent from the following description of the preferred embodiments thereof, and from the claims.

## Description of the Preferred Embodiments

The drawing will first briefly be described.

### Drawing

Fig. 1 is a diagrammatic representation of a hammerhead motif ribozyme showing stems I, II and III (marked (I), (II) and (III) respectively) interacting with a target region.. The 5' and 3' ends of both ribozyme and target are shown. Dashes indicate base-paired nucleotides.

### Target Sites

Ribozymes targeting selected regions of mRNA associated with a selected disease are chosen to cleave the target RNA in a manner which preferably inhibits translation of the RNA. Genes are selected such that inhibition of translation will preferably inhibit cell replication, e.g., by inhibiting production of a necessary protein. Selection of effective target sites within these critical regions of mRNA entails testing the accessibility of the target RNA to hybridization with various oligonucleotide probes. These studies can be performed using RNA probes and assaying accessibility by cleaving the hybrid molecule with RNaseH (see below). Alternatively, such a study can use ribozyme probes designed from secondary structure, predictions of the mRNAs, and assaying cleavage products, by polyacrylamide gel electrophoresis (PAGE), to detect the presence of cleaved and uncleaved molecules.

The following is but one example of a method by which suitable target sites can be identified and is not limiting in this invention. Generally, the method involves identifying potential cleavage sites for a hammerhead ribozyme, and then testing each of these sites to determine their suitability as targets by ensuring that secondary structure formation is minimal.

The mRNA sequences are compared in an appropriate target region. Putative ribozyme cleavage sites are found. These sites represent the preferable sites for hammerhead ribozyme cleavage within these target mRNAs.

Short RNA substrates corresponding to each of the mRNA sites are designed. Each substrate is composed of two to three nucleotides at the 5' and 3' ends that will not base pair with a corresponding ribozyme recognition region. The unpaired regions flanked a central region of 12-14 nucleotides to which complementary arms in the ribozyme are designed.

The structure of each substrate sequence is predicted using a standard PC fold computer program. Sequences which give a positive free energy of binding are accepted. Sequences which give a negative free energy are modified by trimming one or two bases from each of the ends. If the modified sequences are still predicted to have a strong secondary structure, they are rejected.

After substrates are chosen, ribozymes are designed to each of the RNA substrates. Ribozyme folding is also analyzed using PC fold.

Ribozyme molecules are sought which form hammerhead motif stem II (see Fig. 1) regions and contain flanking arms which are devoid of intramolecular base pairing. Often the ribozymes are modified by trimming a base from the ends of the ribozyme, or by introducing additional base pairs in stem II to achieve the desired fold. Ribozymes with incorrect folding are rejected. After substrate/ribozyme pairs are found to contain correct intramolecular structures, the molecules are folded together to predict intermolecular interactions. A schematic representation of a ribozyme with its coordinate base pairing to its cognate target sequence is shown in Fig. 1.

Those targets thought to be useful as ribozyme targets can be tested to determine accessibility to nucleic acid probes in a ribonuclease H assay (see below). This assay provides a quick test of the use of the target site without requiring synthesis of a ribozyme. It can be used to screen for sites most suited for ribozyme attack.

<u>Synthesis of Ribozymes</u>

Ribozymes useful' in this invention can be produced by gene transcription as described by Cech, *supra,* or by chemical synthesis. Chemical synthesis of RNA is similar to that for DNA synthesis. The additional 2'-OH group in RNA, however, requires a different protecting group strategy to deal with selective 3'-5' internucleotide bond formation, and with RNA susceptibility to degradation in the presence of bases. The recently developed method of RNA synthesis utilizing the t-butyldimethylsilyl group for the protection of the 2' hydroxyl is the most reliable method for synthesis of ribozymes. The method reproducibly yields RNA with the correct 3'-5' internucleotide linkages, with average coupling yields in excess of 99%, and requires only a two-step deprotection of the polymer.

A method, based upon H-phosphonate chemistry of phosphoroamidites gives a relatively lower coupling efficiency than a method based upon phosphoroamidite chemistry. This is a problem for synthesis of DNA as well. A promising approach to scale-up of automatic oligonucleotide synthesis has been described recently for the H-phosphonates. A combination of a proper coupling time and additional capping of "failure" sequences gave high yields in the synthesis of oligodeoxynucleotides in scales in the range of 14 µmoles with as little as 2 equivalents of a monomer in the coupling step. Another alternative approach is to use soluble polymeric supports (<u>e.g.</u>, polyethylene glycols), instead of the conventional solid supports. This method can yield short oligonucleotides in hundred milligram quantities per batch utilizing about 3 equivalents of a monomer in a coupling step.

Various modifications to ribozyme structure can be made to enhance the utility of ribozymes. Such modifications will enhance shelf-life, half-life *in vitro,* stability, and ease of introduction of such ribozymes to the target site, <u>e.g.</u>, to enhance penetration of cellular membranes, and confer the ability to recognize and bind to targeted cells.

Exogenous delivery of ribozymes benefits from chemical modification of the backbone, <u>e.g.</u>, by the overall negative charge of the ribozyme molecule being reduced to facilitate diffusion across the cell membrane. The present strategies for reducing the oligonucleotide charge include: modification of internucleotide linkages by methylphosphonates, use of phosphoramidites, linking oligonucleotides to positively charged molecules, and creating complex packages composed of oligonucleotides, lipids and specific receptors or effectors for targeted cells. Examples of such modifications include sulfur-containing ribozymes containing phosphorothioates and phosphorodithioates as internucleotide linkages in RNA. Synthesis of such sulfur-modified ribozymes is achieved by use of the sulfur-transfer reagent, $^{3}H$-1,2-benzenedithiol-3-one 1,1-dioxide. Ribozymes may also contain ribose modified ribonucleotides. Pyrimidine analogues are prepared from uridine using a procedure employing diethylamino sulphur trifluoride (DAST) as a starting reagent. Ribozymes can also be either electrostatically or covalently attached to polymeric cations for the purpose of reducing charge. The polymer can be attached to the ribozyme by simply converting the 3'-end to a ribonucleoside dialdehyde which is obtained by a periodate cleavage of the terminal 2',3'-cis diol system. Depending on the specific requirements for delivery systems, other possible modifications may include different linker arms containing carboxyl, amino or thiol functionalities. Yet further examples include use of methylphosphonates and 2'-O-methylribose and 5' or 3' capping or blocking with $m_7$GpppG or $m_3^{2,2,7}$GpppG.

For example, a kinased ribozyme is contacted with guanosine triphosphate and guanyltransferase to add an $m^3$G cap to the ribozyme. After such synthesis, the ribozyme can be gel purified using standard procedure. To ensure that the ribozyme has the desired activity, it may be tested with and without the 5' cap using standard procedures to assay both its enzymatic activity and its stability.

Synthetic ribozymes, including those containing various modifiers, can be purified by high pressure liquid chromatography (HPLC). Other liquid chromatography techniques, employing reverse phase columns and anion exchangers on silica and polymeric supports may also be used.

There follows an example of the synthesis of one ribozyme. A solid phase phosphoramidite chemistry is employed. Monomers used are 2'-*tert*-butyl-dimethylsilyl cyanoethylphosphoramidities of uridine, N-benzoyl-cytosine, N-phenoxyacetyl adenosine and guanosine (Glen Research, Sterling, VA). Solid phase synthesis is carried out on either an ABI 394 or 380B DNA/RNA synthesizer using the standard protocol provided with each machine. The only exception is that the coupling step is increased from 10 to 12 minutes. The phosphoramidite concentration is 0.1 M. Synthesis is done on a 1 μmole scale using a 1 μmole RNA reaction column (Glen Research). The average coupling efficiencies are between 97% and 98% for the 394 model, and between 97% and 99% for the 380B model, as determined by a calorimetric measurement of the released trityl cation.

Blocked ribozymes are cleaved from the solid support (e.g., CPG), and the bases and diphosphoester moiety deprotected in a sterile vial by dry ethanolic ammonia (2 mL) at 55°C for 16 hours. The reaction mixture is cooled on dry ice. Later, the cold liquid is transferred into a sterile screw cap vial and lyophilized.

To remove the 2'-*tert*-butyl-dimethylsilyl groups from the ribozyme, the residue is suspended in 1 M tetra-n-butylammoniumfluoride in dry THF (TBAF), using a 20 fold excess of the reagent for every silyl group, for 16 hours at ambient temperature (about 15-25°C). The reaction is quenched by adding an equal volume of sterile 1 M triethylamine acetate, pH 6.5. The sample is cooled and concentrated on a SpeedVac to half the initial volume.

The ribozymes are purified in two steps by HPLC on a C4 300 Å 5 mm DeltaPak column in an acetonitrile gradient.

The first step, or "trityl on" step, is a separation of 5'-DMT-protected ribozyme(s) from failure sequences lacking a 5'-DMT group. Solvents used for this step are: A (0.1 M triethylammonium acetate, pH 6.8) and B (acetonitrile). The elution profile is: 20% B for 10 minutes, followed by a linear gradient of 20% B to 50% B over 50 minutes, 50% B for 10 minutes, a linear gradient of 50% B to 100% B over 10 minutes, and a linear gradient of 100% B to 0% B over 10 minutes.

The second step is a purification of a completely deblocked ribozyme by a treatment of 2% trifluoroacetic acid on a C4 300 Å 5 mm DeltaPak column in an acetonitrile gradient. Solvents used for this second step are: A (0.1 M triethylammonium acetate, pH 6.8) and B (80% acetonitrile, 0.1 M triethylammonium acetate, pH 6.8). The elution profile is: 5% B for 5 minutes, a linear gradient of 5% B to 15% B over 60 minutes, 15% B for 10 minutes, and a linear gradient of 15% B to 0% B over 10 minutes.

The fraction containing ribozyme is cooled and lyophilized on a SpeedVac. Solid residue is dissolved in a minimum amount of ethanol and sodium perchlorate in acetone. The ribozyme is collected by centrifugation, washed three times with acetone, and lyophilized.

Expression Vector

While synthetic ribozymes are preferred in this invention, those produced by expression vectors can also be used. In designing a suitable ribozyme expression vector the following factors are important to consider. The final ribozyme must be kept as small as possible to minimize unwanted secondary structure within the ribozyme. A promoter (e.g., the human, cytomegalovirus immediate early promoter or human beta actin promoter) should be chosen to be a relatively strong promoter, and expressible both *in vitro* and *in vivo* (e.g., the human cytomegalovirus immediate early promoter or human beta actin promoter). Such a promoter should express the ribozyme at a level suitable to effect production of enough ribozyme to destroy a target RNA, but not at too high a level to prevent other cellular activities from occurring (unless cell death itself is desired).

A hairpin at the 5' end of the ribozyme is useful to ensure that the required transcription initiation sequence (GG or GGG or GGGAG) does not bind to some other part of the ribozyme and thus affect regulation of the transcription process. The 5' hairpin is also useful to protect the ribozyme from 5'-3' exonucleases. A selected hairpin at the 3' end of the ribozyme gene is useful since it acts as a transcription termination signal, and protects the ribozyme from 3'-5' exonuclease activity. One example of a known termination signal is that present on the T7 RNA polymerase system.

This signal is about 30 nucleotides in length. Other 3' hairpins of shorter length can be used to provide good termination and RNA stability. Such hairpins can be inserted within the vector sequences to allow standard ribozymes to be placed in an appropriate orientation and expressed with such sequences attached.

Poly(A) tails are also useful to protect the 3' end of the ribozyme. These can be provided by either including a poly(A) signal site in the expression vector (to signal a cell to add the poly(A) tail *in vivo*), or by introducing a poly (A) sequence directly into the expression vector. In the first approach the signal must be located to prevent unwanted secondary structure formation with other parts of the ribozyme. In the second approach, the poly(A) stretch may reduce in size over time when expressed *in vivo*, and thus the vector may need to be checked over time. Care must be taken in addition of a poly(A) tail which binds poly(A) binding proteins which prevent the ribozyme from acting.

Ribozyme Testing

Once the desired ribozymes are selected, synthesized and purified, they are tested in kinetic and other experiments to determine their utility. An example of such a procedure is provided below.

Preparation of Ribozyme

Crude synthetic ribozyme (typically 350 μg at a time) is purified by separation on a 15% denaturing polyacrylamide gel (0.75 mm thick, 40 cm long) and visualized by UV shadowing. Once excised, gel slices containing full length ribozyme are soaked in 5 ml gel elution buffer (0.5 M $NH_4OAc$, 1 mM EDTA) overnight with shaking at 4°C. The eluent is desalted over a C-18 matrix (Sep-Pak cartridges, Millipore, Milford, MA) and vacuum dried. The dried RNA is resuspended in 50-100 μl TE (TRIS 10 mM, EDTA 1 mM, pH 7.2). An aliquot of this solution is diluted 100-fold into 1 ml TE, half of which is used to spectrophotometrically quantitate the ribozyme solution. The concentration of this dilute stock is typically 150-800 nM. Purity of the ribozyme is confirmed by the presence of a single band on a denaturing polyacrylamide gel.

A ribozyme may advantageously be synthesized in two or more portions. Each portion of a ribozyme will generally have only limited or no enzymatic activity, and the activity will increase substantially (by at least 5-10 fold) when all portions are ligated (or otherwise juxtaposed) together. A specific example of hammerhead ribozyme synthesis is provided below.

The method involves synthesis of two (or more) shorter "half" ribozymes and ligation of them together using T4 RNA ligase. For example, to make a 34 mer ribozyme, two 17 mers are synthesized, one is phosphorylated, and both are gel purified. These purified 17 mers are then annealed to a DNA splint strand complementary to the two 17 mers. (Such a splint is not always necessary.) This DNA splint has a sequence designed to locate the two 17 mer portions with one end of each adjacent each other. The juxtaposed RNA molecules are then treated with T4 RNA ligase in the presence of ATP. The 34 mer RNA so formed is then HPLC purified.

Preparation of Substrates

Approximately 10-30 pmoles of unpurified substrate is radioactively 5' end-labeled with T4 polynucleotide kiriase using 25 pmoles of. [$\gamma$-$^{32}$P] ATP. The entire labeling mix is separated on a 20% denaturing polyacrylamide gel and visualized by autoradiography. The full length band is excised and soaked overnight at 4°C in 100 μl of TE (10 mM Tris-HCl pH 7.6, 0.1 mM EDTA).

Kinetic Reactions

For reactions using short substrates (between 8 and 16 bases) a substrate solution is made 1X in assay buffer (75 mM Tris-HCl, pH 7.6; 0.1 mM EDTA, 10 mM $MgCl_2$) such that the concentration of substrate is less than 1 nM. A ribozyme solution (typically 20 nM) is made 1X in assay buffer and four dilutions are made using 1X assay buffer. Fifteen μl of each ribozyme dilution (i.e., 20, 16, 12, 8 and 4 nM) is placed in a separate tube. These tubes and the substratetube are preincubated at 37°C for at least five minutes.

The reaction is started by mixing 15 μl of substrate into each ribozyme tube by rapid pipetting (note that final ribozyme concentrations are 10, 8, 6, 4, 2 nM). Five μl aliquots are removed at 15 or 30 second intervals and quenched with 5 μl stop solution (95% formamide, 20 mM EDTA xylene cyanol, and bromphenol blue dyes). Following the final ribozyme time point, an aliquot of the remaining substrate is removed as a zero ribozyme control.

The samples are separated on either 15% or 20% polyacrylamide gels. Each gel is visualized and quantitated with an Ambis beta scanner (Ambis Systems, San Diego, CA).

For the most active ribozymes, kinetic analyses are performed in substrate excess to determine $K_m$ and $K_{cat}$ values.

For kinetic reactions with long RNA substrates (greater than 15 bases in length) the substrates are prepared by transcription using T7 RNA polymerase and defined templates containing a T7 promoter, and DNA encoding appropriate nucleotides of the target RNA. The substrate solution is made 1X in assay buffer (75 mM Tris-HCl, pH 7.6; 0.1 mM EDTA; 10 mM $MgCl_2$) and contains 58 nanomolar concentration of the long RNA molecules. The reaction is started by addition of gel purified ribozymes to 1 µM concentration. Aliquots are removed at 20, 40, 60, 80 and 100 minutes, then quenched by the addition of 5 µl stop solution. Cleavage products are separated using denaturing PAGE. The bands are visualized and quantitated with an Ambis beta scanner.

Kinetic Analysis

A simple reaction mechanism for ribozyme-mediated cleavage is:

$$R + S \underset{k_{-1}}{\overset{k_1}{\rightleftharpoons}} [R:S] \overset{k_2}{\rightleftharpoons} [R:P] \quad \boxed{\rightarrow R + P}$$

where R = ribozyme, S = substrate, and P = products. The boxed step is important only in substrate excess. Because ribozyme concentration is in excess over substrate concentration, the concentration of the ribozyme-substrate complex ([R:S]) is constant over time except during the very brief time when the complex is being initially formed, i.e., :

$$\frac{d[R:S]}{dt} = 0$$

where t = time, and thus:

$$(R)\ (S)k_1 = (RS)\ (k_2 + k_1).$$

The rate of the reaction is the rate of disappearance of substrate with time:

$$\text{Rate} = \frac{-d(S)}{dt} = k_2(RS)$$

Substituting these expressions:

$$(R)\ (S)k_1 = 1/k_2\ \frac{-d(S)}{dt}\ (k_2 + k_1)$$

or:

$$\frac{-d(S)}{S} = \frac{k_1 k_2}{(k_2 + k_1)}\ (R)\ dt$$

Integrating this expression with respect to time yields:

$$-\ln \frac{S}{S_0} = \frac{k_1 k_2}{(k_2 + k_1)}\ (R)\ t$$

where $S_0$ = initial substrate. Therefore, a plot of the negative log of fraction substrate uncut versus time (in minutes) yields a straight line with slope:

$$\text{slope} = \frac{k_1 k_2}{(k_2 + k_1)} (R) = k_{obs}$$

where $k_{obs}$ = observed rate constant. A plot of slope ($k_{obs}$) versus ribozyme concentration yields a straight line with a slope which is:

$$\text{slope} = \frac{k_1 k_2}{(k_2 + k_1)}$$

which is

$$\frac{k_{cat}}{K_m}$$

Using these equations the data obtained from the kinetic experiments provides the necessary information to determine which ribozyme tested is most useful, or active. Such ribozymes can be selected and tested in *in vivo* or *ex vivo* systems.

Liposome Preparation

Lipid molecules are dissolved in a volatile organic solvent ($CHCl_3$, methanol, diethylether, ethanol, etc.). The organic solvent is removed by evaporation. The lipid is hydrated into suspension with 0.1x phosphate buffered saline (PBS), then freeze-thawed 3x using liquid nitrogen and incubation at room temperature. The suspension is extruded sequentially through a 0.4 µm, 0.2 µm and 0.1 µm polycarbonate filters at maximum pressure of 800 psi. The ribozyme is mixed with the extruded liposome suspension and lyophilized to dryness. The lipid/ribozyme powder is rehydrated with water to one-tenth the original volume. The suspension is diluted to the minimum volume required for extrusion (0.4 ml for 1.5 ml barrel and 1.5 ml for 10 ml barrel) with 1xPBS and re-extruded through 0.4 µm, 0.2 µm, 0.1 µm polycarbonate filters. The liposome entrapped ribozyme is separated from untrapped ribozyme by gel filtration chromatography (SEPHAROSE CL-4B, BIOGEL A5M). The liposome extractions are pooled and sterilized by filtration through a 0.2 µm filter. The free ribozyme is pooled and recovered by ethanol precipitation. The liposome concentration is determined by incorporation of a radioactive lipid. The ribozyme concentration is determined by labeling with $^{32}P$. Rossi et al., 1992, *supra* (and references cited therein) describe other methods suitable for preparation of liposomes.

Examples of other useful liposome preparations which display similar degrees of uptake of both a radioactive lipid marker and an entrapped fluorophore by Vero cells showed different fluorescent staining patterns. Specifically, liposomes composed of DPPG/DPPC/Cholesterol (in a ratio of: 50/17/33) gave a punctate pattern of fluorescence, while DOPE/Egg PC/Cholesterol (30/37/33) gave a diffuse, homogeneous pattern of fluorescence in the cytoplasm. Cell fractionation showed that 80% of the entrapped contents from the DPPG/DPPC/Cholesterol formulation was localized in the membrane fraction, whereas the DOPE/Egg PC/Cholesterol formulation was localized in the cytoplasm. Further characterization of the latter formulation showed that after 3 hours, 70% of the fluorescence was cytoplasmic and 30% was in the membrane. After 24 hours, uptake had increased 5 fold and the liposome contents were distributed 50/50 between the cytoplasmic and membrane fractions.

Liposomes containing 15 ribozymes ($^{32}P$-labeled) targeted to the HSV ICP4 mRNA were prepared and incubated with the cells. After 24 hours, 25% of the liposome dose was taken up with approximately 60,000 liposomes per cell. Thirty percent of the delivered ribozyme was intact after 24 hours. Cell fractionation studies showed 40% of the intact ribozyme to be in the membrane fraction and 52% of the intact ribozyme to be in the cytoplasmic fraction.

In Vivo Assay

The efficacy of action of a chosen ribozyme may be tested *in vivo* using standard procedures in transformed cells or animals which express the target mRNA.

Ribonuclease Protection Assay

The accumulation of target mRNA in cells or the cleavage of the RNA by ribozymes or RNaseH (*in vitro* or *in vivo*) can be quantified using an RNase protection assay.

In this method, antisense riboprobes are transcribed from template DNA using T7 RNA polymerase (U.S. Biochemical) in 20 µl reactions containing 1X transcription buffer (supplied by the manufacturer), 0.2 mM ATP, GTP and UTP, 1 U/µl pancreatic RNase inhibitor (Boehringer Mannheim Biochemicals) and 200 µCi $^{32}$P-labeled CTP (800 Ci/mmol, New England Nuclear) for 1 hour at 37°C. Template DNA is digested with 1 U RNase-free DNaseI (U.S. Biochemical, Cleveland, OH) at 37°C for 15 minutes and unincorporated nucleotides removed by G-50 SEPHADEX spin chromatography.

In a manner similar to the transcription of antisense probe, the target RNA can be transcribed. *in vitro* using a suitable DNA template. The transcript is purified by standard methods and digested with ribozyme at 37°C according to methods described later.

Alternatively, afflicted (mRNA-expressing) cells are harvested into 1 ml of PBS, transferred to a 1.5, ml EPPENDORF tube, pelleted for 30 seconds at low speed in a microcentrifuge, and lysed in 70 µl of hybridization buffer (4 M guanidine isothiocyanate, 0.1% sarcosyl, 25 mM sodium citrate, pH 7.5). Cell lysate (45 µl) or defined amounts of *in vitro* transcript (also in hybridization buffer) is then combined with 5 µl of hybridization buffer containing 5 x $10^5$ cpm of each antisense riboprobe in 0.5 ml Eppendorf tubes, overlaid with 25 µl mineral oil, and hybridization accomplished by heating overnight at 55°C. The hybridization reactions are diluted into 0.5 ml RNase solution (20 U/ml RNaseA, 2 U/ml RNaseT1, 10 U/ml RNase-free DNaseI in 0.4 M NaCl), heated for 30 minutes at 37°C, and 10 µl of 20% SDS and 10 µl of Proteinase K (10 mg/ml) added, followed by an additional 30 minutes incubation at 37°C. Hybrids are partially purified by extraction with 0.5 ml of a 1:1 mixture of phenol/chloroform; aqueous phases are combined with 0.5 ml isopropanol, and RNase-resistant hybrids pelleted for 10 minutes at room temperature (about 20°C) in a microcentrifuge. Pellets are dissolved in 10 µl loading buffer (95% formamide, 1X TBE, 0.1% bromophenol blue, 0.1% xylene cylanol), heated to 95°C for five minutes, cooled on ice, and analyzed on 4% polyacrylamide/7 M urea gels under denaturing conditions.

Ribozyme Stability

The chosen ribozyme can be tested to determine its stability, and thus its potential utility. Such a test can also be used to determine the effect of various chemical modifications (e.g., addition of a poly(A) tail) on the ribozyme stability and thus aid selection of a more stable ribozyme. For example, a reaction mixture contains 1 to 5 pmoles of 5' (kinased) and/or 3' labeled ribozyme, 15 µg of cytosolic extract and 2.5 mM $MgCl_2$ in a total volume of 100 µl. The reaction is incubated at 37°C. Eight µl aliquots are taken at timed intervals and mixed with 8 µl of a stop mix (20 mM EDTA, 95% formamide). Samples are separated on a 15% acrylamide sequencing gel, exposed to film, and scanned with an Ambis.

A 3'-labeled ribozyme can be formed by incorporation of the $^{32}$P-labeled cordycepin at the 3' OH using poly (A) polymerase. For example, the poly(A) polymerase reaction contains 40 mM Tris, pH 8, 10 mM $MgCl_2$, 250 mM NaCl, 2.5 mM $MnCl_2$,; 3 µl $^{32}$P cordycepin, 500 Ci/mM; and 6 units poly(A) polymerase in a total volume of 50 µl. The reaction mixture is incubated for 30 minutes at 37°C.

Effect of Base Substitution Upon Ribozyme Activity

To determine which primary structural characteristics could change ribozyme cleavage of substrate, minor base changes can be made in the substrate cleavage region recognized by a specific ribozyme. For example, the substrate sequences can be changed at the central "C" nucleotide, changing the cleavage site from a GUC to a GUA motif. The $K_{cat}/K_m$ values for cleavage using each substrate are then analyzed to determine if such a change increases ribozyme cleavage rates. Similar experiments can be performed to address the effects of changing bases complementary to the ribozyme binding arms. Changes predicted to maintain strong binding to the complementary substrate are preferred. Minor changes in nucleotide content can alter ribozyme/substrate interactions in ways which are unpredictable based upon binding strength alone. Structures in the catalytic core region of the ribozyme recognize trivial changes in either substrate structure or the three dimensional structure of the ribozyme/substrate complex.

To begin optimizing ribozyme design, the cleavage rates of ribozymes containing varied arm lengths, but targeted to the same length of short RNA substrate can be tested. Minimal arm lengths are required and effective cleavage varies with ribozyme/substrate combinations.

The cleavage activity of selected ribozymes can be assessed using target mRNA substrates. The assays are performed in ribozyme excess and approximate $K_{cat}/K_{min}$ values obtained. Comparison of values obtained with short and long substrates indicates utility *in vivo* of a ribozyme.

Intracellular Stability of Liposome-delivered Ribozymes

To test the stability of a chosen ribozyme *in vivo* the following test is useful. Ribozymes are $^{32}$P-end labeled,

entrapped in liposomes and delivered to target mRNA-containing cells for three hours. The cells are fractionated and ribozyme is purified by phenol/chloroform extraction. Alternatively, cells (1x$10^7$, T-175 flask) are scraped from the surface of the flask and washed twice with cold PBS. The cells are homogenized by douncing 35 times in 4 ml of TSE (10 mM Tris, pH 7.4, 0.25 M Sucrose, mM EDTA). Nuclei are pelleted at 100xg for 10 minutes. Subcellular organelles (the membrane fraction) are pelleted at 200,000kg for two hours using an SW60 rotor. The pellet is resuspended in 1 ml of H buffer (0.25 M Sucrose, 50 mM HEPES, pH 7.4). The supernatant contains the cytoplasmic fraction (in approximately 3.7 ml). The nuclear pellet is resuspended in 1 ml of 65% sucrose in TM (50 mM Tris, pH 7.4, 2.5 mM $MgCl_2$) and banded on a sucrose step gradient (1 ml nuclei in 65% sucrose TM, 1 ml 60% sucrose TM, 1 ml 55% sucrose TM, 50% sucrose TM, 300 μl 25% sucrose TM) for one hour at 37,000xg with an SW60 rotor. The nuclear band is harvested and diluted to 10% sucrose with TM buffer. Nuclei are pelleted at 37,000xg using an SW60 rotor for 15 minutes and the pellet resuspended in 1 ml of TM buffer. Aliquots are size fractionated on denaturing polyacrylamide gels and. the intracellular localization determined. By comparison to the migration rate of newly synthesized ribozyme, the various fractions containing intact ribozyme can be determined.

To investigate modifications which would lengthen the half-life of ribozyme molecules intracellularly, the cells may be fractioned as above and the purity of each fraction assessed by assaying enzyme activity known to exist in that fraction.

The various cell fractions are frozen at -70°C and used to determine relative nuclease resistances of modified ribozyme molecules. Ribozyme molecules may be synthesized with 5 phosphorothioate (ps), or 2'-Omethyl (2' -OMe) modifications at each end of the molecule. These molecules and a phosphodiester version of the ribozyme are end-labeled with $^{32}P$ and ATP using T4 polynucleotide kinase. Equal concentrations are added to the cell cytoplasmic extracts and aliquots of each taken at 10 minute intervals. The samples are size fractionated by denaturing PAGE and relative rates of nuclease resistance analyzed by scanning the gel with an Ambis β-scanner. The results show whether the ribozymes are digested by the cytoplasmic extract, and which versions are relatively more nuclease resistant. Modified ribozymes generally maintain 8.0-90% of the catalytic activity of the native ribozyme when short RNA substrates are employed.

Unlabeled, 5' end-labeled or 3' end-labeled ribozymes can be used in the assays. These experiments can also be performed with human cell extracts to verify the observations.

In one example, Vero or HeLa cells were grown to 90-95% confluency in 175 $cm^2$ tissue culture flasks, scraped into 10 ml of cold phosphate buffered saline (PBS), then washed once in 10 ml of cold PBS and once in 10 ml of cold TSE (10 mM Tris, pH 7.4; 0.25 M sucrose; 1 mM EDTA); The cell pellets were resuspended in 4 ml of TSE, dounced 35x on ice, and the released nuclei pelleted by centrifugation at 1000g for 10 minutes. The nuclear pellet was resuspended in 1 ml of 65% sucrose TM (50 mM Tris, pH 7.4; 2.5 mM $MgCl_2$) and transferred to Beckman ultra-clear tubes. The following sucrose TM solutions were layered on top of the sample: 1 ml 60%, 1 ml 55%, and 25% sucrose to the top of the tube. Gradients were spun in an SW60 rotor at 37,000g for 1 hour. HeLa nuclei banded at the 55-60% sucrose boundary and Vero nuclei banded at the 50-55% sucrose boundary. Nuclear bands were harvested, diluted to 10% sucrose with TM buffer, and pelleted by centrifugation at 37,000g for 15 minutes using an SW60 rotor. The nuclear pellet was resuspended in 1 ml of TM buffer. Subcellular organelles and membrane components in the post nuclear supernatant were separated from the cytoplasmic fraction by centrifugation at 200,000g for 2 hours in an SW60 rotor. The pellet contained the membrane fraction, which was resuspended in 1 ml of H buffer (0.25 M sucrose; 50 mM HEPES, pH 7.4), and the supernatant contained the cytoplasmic fraction.

Purity of the various fractions was assessed using enzymatic markers specific for the cytoplasmic and membranous fractions. Three enzyme markers for the membranous fraction were used; hexosaminidase and β-glucocerebrosidase are localized in lysosomes, while alkaline phosphodiesterase is specific to endosomes. Specifically, the assays were as follows:

For N-acetyl-beta-hexosaminidase, the reaction mixture contained 0.3 mg/ml 4-methylumbelliferyl-N-acetylglucosaminide; 20 mM sodium citrate; pH 4.5; 0.01% Triton X-100; and 100 μl of sample in a final volume of 500 μl (Harding et al., 64 Cell 393, 1991). The reactions were incubated at 37°C for 1 hour and stopped by the addition of 1.5 ml of stop buffer (0.13 M glycine, 0.07 M NaCl, 0.08 M sodium carbonate, pH 10.6). The reaction product was quantitated in a Hitachi F-4010 fluorescence spectrophotometer by excitation of the fluorophore at 360 nm and analysis of the emission at 448 nm.

For Alkaline Phosphodiesterase, the assay medium contained 25 mM CAPS (3-(Cyclohexylamino)-propanesulfonic acid), pH 10.6; 0.05% Triton X-100; 15 mM $MgCl_2$; 1.25 mg/ml Thymidine-5'-monophosphate-p-nitrophenyl ester; and 100 μl of sample in a total reaction volume of 200 μl. The reactions were incubated at 37°C for 2 hours, then diluted to 1 ml with $H_2O$ and the absorbance was measured at 400 nm (Razell and Khorana, 234 J. Biol. Chem. 739, 1959).

For β-glucocerebrosidase, the reaction contained 85 nM sodium citrate, pH 5.9; 0.12% Triton X-100; 0.1% sodium taurocholate; 5 mM 4-methylumbelliferyl β-D-glucopyranoside; and 125 μl of sample in a total volume of 250 μl (Kennedy and Cooper, 252 Biochem. J. 739, 1988). The reaction was incubated at 37°C for 1 hour and stopped by the addition of 0.75 ml of stop buffer. Product formation was measured in a fluorescence spectrophotometer by using an excitation wavelength of 360 nm and analysis of the emission at 448 nm.

The cytoplasmic enzyme marker, lactate dehydrogenase, was assayed in an assay mixture containing 0.2 M Tris; pH 7.4; 0.22 mM NADH; 1 mM sodium pyruvate; and 50 µl of sample in a final volume of 1.05 ml. Enzyme levels were determined by decreased absorbency at 350 nm resulting from the oxidation of NADH at room temperature (Silverstein and Boyer, 239 J. Biol. Chem. 3901, 1964).

Lactate dehydrogenase was found predominantly in the cytoplasmic fractions of both Vero and HeLa cells, while β-glucocerebrosidase and alkaline phosphodiesterase were found almost exclusively in the membranous fractions. The hexosaminidase activity in Vero cell fractions was concentrated in the membranous fraction (70%) with about 20% in the cytoplasmic fraction. The isolation of enzyme markers with the appropriate cellular compartment demonstrated that cytoplasmic, membranous and nuclear fractions can be isolated with minimal intercompartmental contamination using this fractionation scheme.

Nuclease Stability of Ribozymes and mRNA

The simplest and most sensitive way to monitor nuclease activity in cell fractions is to use end-labeled oligonucleotides. However, high levels of phosphatase activity in some biological extracts gives ambiguous results in nuclease experiments when $^{32}$P-5'-end-labeled oligonucleotides are used as substrates. To determine the phosphatase activity in the extracts, cellular fractions were incubated with cold ribozymes and trace amounts of 5'-end-labeled ribozyme in the presence of 1 mM $Mg^{+2}$ (or $Zn^{+2}$ with HeLa cytoplasmic extracts) to optimize digestion. After polyacrylamide gel electrophoresis of samples, digestion of the oligonucleotide was assessed both by staining and by autoradiography.

Specifically, the basic oligonucleotide digestion reaction contained substrate nucleic acid (an RNA oligonucleotide of 36 nucleotides) and cell fraction extract in a total volume of 100 µl. Aliquots (7 µl) were taken after various periods of incubation at 37°C. and added to 7 µl of gel loading buffer (95% formamide, 0.1% bromophenol blue, 0.1% xylene cyanol, and 20 mM EDTA). The samples were separated by electrophoresis on a 7 M urea, 20% polyacrylamide gel. Intact ribozymes were visualized either by staining with Stains-all (United States Biochemical, Cleveland, OH), or autoradiography of $^{32}$P-labeled ribozyme. The stained gels and X-ray films were scanned on a Bio 5000 density scanner (U.S. Biochemical). Ribozymes were 5' end-labeled with T4 polynucleotide kinase (U.S. Biochemical) using 10 µCi of $^{32}$P γ-ATP (3,000 Ci/mmole, New England Nuclear, Boston, MA), and 20-25 pmoles of ribozymes. The unincorporated nucleotides were separated from the product by G-50 spin chromatography. Nuclease assays contained 1-2 pmoles of $^{32}$P-labeled ribozyme. All oligonucleotides were synthesized on an Applied Biosystems 394 DNA/RNA synthesizer (Applied Biosystems Inc., Foster City, CA) according to manufacturer's protocols. The nuclear fractions were resuspended in a buffer containing 2.5 mM $MgCl_2$. Experiments involving the nuclear fractions were performed in the presence of 1 mM $Mg^{+2}$, or in combination with 1 mM $Mn^{+2}$, $Ca^{+2}$, or $Zn^{+2}$.

To measure the stability of mRNA, Vero cells were infected with herpes simplex virus (HSV) at a M.O.I. of 5 and total RNA was extracted (Chomczynski and Sacchi, 162 Anal. Biochem. 156, 1987). An RNase protection assay was used to detect mRNA after incubation of total infected cellular RNA in cytoplasmic extracts. RNA probes were produced from PCR-amplified template DNA using T7 RNA polymerase (U.S. Biochemical) in the presence of $^{32}$P α-CTP (3,000 Ci/mmole, New England Nuclear, Boston, MA). Template DNA was inactivated with 1 unit of RNase-free DNaseI for 15 minutes at 37°C. Unincorporated nucleotides were removed by G-50 spin chromatography. Samples (6 µl) were taken from the nuclease assays after various periods of incubation at 37°C, added to 40 µl of 4 M GUSCN buffer (4 M guanidinium thiocyanate; 25 mM sodium citrate, pH 7; 0.5% sarcosyl; and 0.1 M 2-mercaptoethanol); and 5 µl of $^{32}$P-labeled RNA probe ($5x10^5$ cpm/5 µl, specific activity of $1.8x10^6$ cpm/µg) in 4 M GUSCN buffer. Hybridization reactions were covered with mineral oil and incubated at 55°C for 12-16 hours, after which the hybridization reaction was mixed with 500 µl of RNase buffer (0.4 M NaCl, 20 µg/ml RNaseA, 2 units/ml T1 RNase) and incubated for 30 minutes at 37°C. RNase activity was quenched by incubation with 10 µl of 20% SDS and 10 µl of proteinase K (20 mg/ml), and the RNA was extracted using a phenol/chloroform mixture. The protected RNA fragment was purified by precipitation with an equal volume of isopropanol in the presence of 20 µg of carrier yeast tRNA. The RNA pellets were resuspended in gel loading buffer, heated to 95°C for 5 minutes and separated by electrophoresis on a 5% polyacrylamide, 7 M urea gel. Protected fragments were visualized by autoradiography, and the films were scanned with a Bio 5000 density scanner.

In experiments using these methods, the rate of digestion of ribozymes in Vero cell extracts was similar, demonstrating the lack of significant phosphatase activity in Vero cellular fractions. Similar results were observed with HeLa cellular fractions. In most extracts, ladders of digested fragments were observed; such ladders would not be expected if digestion was an artifact of phosphatase action. Thus, digestion using 5' end-labeled ribozymes is an accurate assessment of nuclease action in cellular extracts.

In other experiments, labeled ribozymes were incubated in various Vero and HeLa cellular fractions. Incubation of ribozymes in either membranous or nuclear fractions resulted in a linear decrease of intact molecules over time. In contrast, no digestion of ribozymes occurred during a 24 hour incubation in Vero cytoplasmic extracts, and HeLa cytoplasmic extracts exhibited a 20-30 minute delay in the onset of RNA digestion. After this refractory period, the rate of digestion was linear but not as rapid as the rates observed in any of the nuclear or membranous fractions.

The effect of four divalent cations ($Mg^{+2}$, $Mn^{+2}$, $Ca^{+2}$, and $Zn^{+2}$) on the nuclease activity of the cellular fractions was assessed. Vero cytoplasmic extracts were stimulated by the addition of 1 mM $Mg^{+2}$ or $Mn^{+2}$, while $Ca^{+2}$. or $Zn^{+2}$ had no effect. Nuclease activity in HeLa cytoplasmic extracts was enhanced only by the addition of 1 mM $Zn^{+2}$. Both Vero and HeLa membranous fractions exhibited maximum nuclease activity with the addition of $Mg^{+2}$ or $Mn^{+2}$ ions, while the addition of $Ca^{+2}$ significantly reduced activity of the HeLa membranous fraction and abolished nuclease activity in the Vero membranous fraction. Addition of $Zn^{+2}$ to both membranous fractions resulted in a loss of all RNase activity. The Vero nuclear extract demonstrated roughly equivalent nuclease activity in the presence of either $Mg^{+2}$ alone or a $Mg^{+2}$ and $Mn^{+2}$ ion combination, less in the presence of $Mg^{+2}$ and $Ca^{+2}$, and no activity in the presence of $Mg^{+2}$ and $Zn^{+2}$. The effects of cation addition were not as dramatic with HeLa nuclear extracts. The nuclease activity of these fractions was greatest in the presence of $Mg^{+2}$ alone or $Mg^{+2}$ and $Ca^{+2}$ and decreased slightly with the addition of $Mn^{+2}$ or $Zn^{+2}$ to the $Mg^{+2}$ present in the extracts.

To verify that nuclease activity was dependent upon added divalent cations, nuclease assays were performed using 1 mM $Mg^{+2}$ in the presence and absence of 20 mM EDTA. For the HeLa cytoplasmic fractions, the $Mg^{+2}$ was replaced with 1 mM $Zn^{+2}$. The presence of 20 mM EDTA completely abolished nuclease activity in the Vero and HeLa cytoplasmic fractions and Vero nuclear fractions. Nuclease activity in the HeLa membranous and nuclear fractions was partially inhibited by the addition of EDTA, while EDTA had no effect on the nuclease activity in the Vero membranous fraction. For comparative purposes, reactions using DNA oligonucleotides were performed using different Vero fractions. All DNase activity in Vero cytoplasmic, membranous, and nuclear fractions was inhibited by 20 mM EDTA.

The stability of RNA oligonucleotides and HSV-1 mRNA were compared in the presence and absence of activity-enhancing divalent cations (1 mM $Mg^{+2}$, Vero cells; 1 mM $Zn^{+2}$, HeLa cells). Total cellular RNA from HSV-1 infected Vero cells (8 mg) and tracer amounts of $^{32}$P-5'-end-labeled RNA oligonucleotides (1 pmole) were incubated with Vero or HeLa cytoplasmic extracts. In the absence of divalent cations, no substantial decrease of intact ribozymes was detected in assays, although mRNA was digested in both Vero and HeLa cytoplasmic extracts. After addition of divalent cations, digestion of ribozymes occurred in both Vero and HeLa cytoplasmic fractions. The rate of ribozyme digestion in HeLa extracts increased to levels similar to those observed with mRNA, while the rate of mRNA digestion remained greater than the rate of ribozyme digestion in Vero cytoplasmic fractions.

Thus, the stability of hammerhead ribozymes were compared in both Vero and HeLa cell cytoplasmic, membranous and nuclear fractions. Vero cytoplasmic and nuclear fractions were found to require $Mg^{+2}$ for optimal nuclease activity, while the membranous fraction was not altered by the addition of divalent cations. HeLa membranous and nuclear fractions were also activated by $Mg^{+2}$, while the cytoplasmic fractions required $Zn^{+2}$ for nuclease activation. Relative stabilities of ribozymes and mRNAs were compared in Vero and HeLa cytoplasmic fractions. In the absence of appropriate divalent cations, little ribozyme digestion was observed in either cytoplasmic preparation while mRNA was rapidly digested. The addition of $Mg^{+2}$ to Vero cytoplasmic extracts and $Zn^{+2}$ to the HeLa cytoplasmic extracts stimulated ribozyme degradation and enhanced mRNA digestion. These data show that the nuclease sensitivity of ribozymes is cell-type specific, varies with the intracellular compartment studied and may not be able to be predicted from studies with mRNA. Notably, however, ribozymes appear stable in such cellular fractions for a period of time potentially sufficient to have a therapeutically useful activity.

Administration of Ribozyme

Selected ribozymes can be administered prophylactically, or to patients having disease conditions, e.g., by exogenous delivery of the ribozyme to a desired tissue by means of an appropriate delivery vehicle, e.g., a liposome, a controlled release vehicle, by use of iontophoresis, electroporation or ion paired molecules, or covalently attached adducts, and other pharmacologically approved methods of delivery. Routes of administration include intramuscular, aerosol, oral (tablet or pill form), topical, systemic, ocular, intraperitoneal and/or intrathecal. Expression vectors for immunization with ribozymes and/or delivery of ribozymes are also suitable.

The specific delivery route of any selected ribozyme will depend on the use of the ribozyme. Generally, a specific delivery program for each ribozyme will focus on unmodified ribozyme uptake with regard to intracellular localization, followed by demonstration of efficacy. Alternatively, delivery to these same cells in an organ or tissue of an animal can be pursued. Uptake studies will include uptake assays to evaluate cellular ribozyme uptake, regardless of the delivery vehicle or strategy. Such assays will also determine the intracellular localization of the ribozyme following uptake, ultimately establishing the requirements for maintenance of steady-state concentrations within the cellular compartment containing the target sequence (nucleus and/or cytoplasm). Efficacy and cytotoxicity can then be tested. Toxicity will not only include cell viability but also cell function.

Some methods of delivery that may be used include:

a. encapsulation in liposomes,
b. transduction by retroviral vectors,
c. conjugation with cholesterol,
d. localization to nuclear compartment utilizing nuclear targeting site found on most nuclear proteins,
e. neutralization of charge of ribozyme by using nucleotide derivatives, and
f. use of blood stem cells to distribute ribozymes throughout the body.

At least three types of delivery strategies are useful in the present invention, including: ribozyme modifications, particle carrier drug delivery vehicles, and retroviral expression vectors. Unmodified ribozymes, like most small molecules, are taken up by cells, albeit slowly. To enhance cellular uptake, the ribozyme may be modified essentially at random, in ways which reduce its charge but maintains specific functional groups. This results in a molecule which is able to diffuse across the cell membrane, thus removing the permeability barrier.

Modification of ribozymes to reduce charge is just one approach to enhance the cellular uptake of these larger molecules. The random approach, however, is not advisable since ribozymes are structurally and functionally more complex than small drug molecules. The structural requirements necessary to maintain ribozyme catalytic activity are well understood by those in the art. These requirements are taken into consideration, when designing modifications to enhance cellular delivery. The modifications are also designed to reduce susceptibility to nuclease degradation. Both of these characteristics should greatly improve the efficacy of the ribozyme. Cellular uptake can be increased by several orders of magnitude without having to alter the phosphodiester linkages necessary for ribozyme cleavage activity.

Chemical modifications of the phosphate backbone will reduce the negative charge allowing free diffusion across the membrane. This principle has been successfully demonstrated for antisense DNA technology. The similarities in chemical composition between DNA and RNA make this a feasible approach. In the body, maintenance of an external concentration will be necessary to drive, the diffusion of the modified ribozyme into the cells of the tissue. Administration routes which allow the diseased tissue to be exposed to a transient high concentration of the drug, which is slowly dissipated by systemic adsorption are preferred. Intravenous administration with a drug carrier designed to increase the circulation half-life of the ribozyme can be used. The size and composition of the drug carrier restricts rapid clearance from the blood stream. The carrier, made to accumulate at the site of infection, can protect the ribozyme from degradative processes.

Drug delivery vehicles are effective for both systemic and topical administration. They can be designed to serve as a slow release reservoir, or to deliver their contents directly to the target cell. An advantage of using direct delivery drug vehicles is that multiple molecules are delivered per uptake. Such vehicles have been shown to increase the circulation half-life of drugs which would otherwise be rapidly cleared from the blood stream. Some examples of such specialized drug delivery vehicles which fall into this category are liposomes, hydrogels, cyclodextrins, biodegradable nanocapsules, and bioadhesive microspheres.

From this category of delivery systems, liposomes are preferred. Liposomes increase intracellular stability, increase uptake efficiency and improve biological activity.

Liposomes are hollow spherical vesicles composed of lipids arranged in a similar fashion as those lipids which make up the cell membrane. They have an internal aqueous space for entrapping water soluble compounds and range in size from 0.05 to several microns in diameter. Several studies have shown that liposomes can deliver RNA to cells and that the RNA remains biologically active.

For example, a liposome delivery vehicle originally designed as a research tool, Lipofectin, has been shown to deliver intact mRNA molecules to cells yielding production of the corresponding protein.

Liposomes offer several advantages: They are non-toxic and biodegradable in composition; they display long circulation half-lives; and recognition molecules can be readily attached to their surface for targeting to tissues. Finally, cost effective manufacture of liposome-based pharmaceuticals, either in a liquid suspension or lyophilized product, has demonstrated the viability of this technology as an acceptable drug delivery system.

Other controlled release drug delivery systems, such as nonoparticles and hydrogels may be potential delivery vehicles for a ribozyme. These carriers have been developed for chemotherapeutic agents and protein-based pharmaceuticals, and consequently, can be adapted for ribozyme delivery.

Topical administration of ribozymes is advantageous since it allows localized concentration at the site of administration with minimal systemic adsorption. This simplifies the delivery strategy of the ribozyme to the disease site and reduces the extent of toxicological characterization. Furthermore, the amount of material to be applied is far less than that required for other administration routes. Effective delivery requires the ribozyme to diffuse into the infected cells. Chemical modification of the ribozyme to neutralize negative charge may be all that is required for penetration. However, in the event that charge neutralization is insufficient, the modified ribozyme can be co-formulated with permeability enhancers, such as Azone or oleic acid, in a liposome. The liposomes can either represent a slow release presentation vehicle in which the modified ribozyme and permeability enhancer transfer from the liposome into the infected cell, or the liposome phospholipids can participate directly with the modified ribozyme and permeability enhancer in facilitating cellular delivery. In some cases, both the ribozyme and permeability enhancer can be formulated into a suppository formulation for slow release.

Ribozymes may also be systemically administered. Systemic absorption refers to the accumulation of drugs in the blood stream followed by distribution throughout the entire body. Administration routes which lead to systemic absorption include:

intravenous, subcutaneous, intraperitoneal, intranasal, intrathecal and ophthalmic. Each of these administration routes expose the ribozyme to an accessible diseased tissue. Subcutaneous administration drains into a localized lymph node which proceeds through the lymphatic network into the circulation. The rate of entry into the circulation has been shown to be a function of molecular weight or size. The use of a liposome or other drug carrier localizes the ribozyme at the lymph node. The ribozyme can be modified to diffuse into the cell, or the liposome can directly participate in the delivery of either the unmodified or modified ribozyme to the cell.

A liposome formulation containing phbsphatidyl-ethanolomidomethylthiosuccinimide which can deliver oligonucleotides to lymphocytes and macrophages is useful for certain conditions. Furthermore, a 200 nm diameter liposome of this composition was internalized as well as 100 nm diameter liposomes. The 200 nm liposomes exhibit a ten-fold greater packaging capacity than the 100 nm liposomes and can accommodate larger molecules such as a ribozyme expression vector. This ribozyme delivery system prevents mRNA expression in afflicted primary immune cells. Whole blood studies show that the formulation is taken up by 90% of the lymphocytes after 8 hours at 37°C. Preliminary biodistribution and pharmacokinetic studies yielded 70% of the injected dose/gm of tissue in the spleen after one hour following intravenous administration.

Liposomes injected intravenously show accumulation in the liver, lung and spleen. The composition and size can be adjusted so that this accumulation represents 30% to 40% of the injected dose. The remaining dose circulates in the blood stream for up to 24 hours.

The chosen method of delivery should result in cytoplasmic accumulation and molecules should have some nuclease-resistance for optimal dosing. Nuclear delivery may be used but is less preferable. Most preferred delivery methods include liposomes (10-400 nm), hydrogels, controlled-release polymers, microinjection or electroporation (for *ex vivo* treatments) and other pharmaceutically applicable vehicles. The dosage will depend upon the disease indication and the route of administration but should be between 100-200 mg/kg of body weight/day. The duration of treatment will extend through the course of the disease symptoms, possibly continuously. The number of doses will depend upon disease delivery vehicle and efficacy data from clinical trials.

Establishment of therapeutic levels of ribozyme within the cell is dependent upon the rate of uptake and degradation. Decreasing the degree of degradation will prolong the intracellular half-life of the ribozyme. Thus, chemically modified ribozymes, e.g., with modification of the phosphate backbone, or capping of the 5' and 3' ends of the ribozyme with nucleotide analogs may require different dosaging. Descriptions of useful systems are provided in the art cited above, all of which is hereby incorporated by reference herein.

Other embodiments are within the following claims.

SEQUENCE LISTING

(1) GENERAL INFORMATION:

(i) APPLICANT:
(A) NAME: Ribozyme Pharmaceuticals, Inc.
(B) STREET: 2950 Wilderness Place
(C) CITY: Boulder
(D) STATE: Colorado
(E) COUNTRY: USA
(F) POSTAL CODE (ZIP): 80301
(G) TELEPHONE: 001-303-449-6500

(ii) TITLE OF INVENTION: Method and Reagent for treatment of animal diseases

(iii) NUMBER OF SEQUENCES: 245

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER:

(2) INFORMATION FOR SEQ ID NO: 1:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO: 1:

CAGCAGAGGA CCAGCUA 17

(2) INFORMATION FOR SEQ ID NO: 2

(i) SEQUENCE CHARACTERISTICS:
(A) LENGTH: 14 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO: 2

GCAACUACAG ACCC 14

(2) INFORMATION FOR SEQ ID NO: 3

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:3

CCCCUGAAAA CAACCCUCAG ACGC 24

(2) INFORMATION FOR SEQ ID NO: 4

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:4

CACAUCCCCU GACAAGCUGC CAGGCAGG 28

(2) INFORMATION FOR SEQ ID NO: 5

(i) SEQUENCE CHARACTERISTICS:
(A) LENGTH: 14 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:5

CACAUACUGA CCCA 14

(2) INFORMATION FOR SEQ ID NO: 6

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:6

CCUCUCUCCC CUGGAAAGG 19

(2) INFORMATION FOR SEQ ID NO: 7

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:7

ACACCAUGAG CACUGAAAGC AUGAUCCGGG AC 32

(2) INFORMATION FOR SEQ ID NO: 8

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:8

GCCCCAGGGC UCCAGGC 17

(2) INFORMATION FOR SEQ ID NO: 9

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:9

GGUGCUUGUU CCUCAGCCUC UUC 23

(2) INFORMATION FOR SEQ ID NO: 10

(i) SEQUENCE CHARACTERISTICS:
(A) LENGTH: 14 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:10

CCACCACGCU CUUC 14

(2) INFORMATION FOR SEQ ID NO: 11

(i) SEQUENCE CHARACTERISTICS:
(A) LENGTH: 13 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:11

AAGAGUUCCC CAG 13

(2) INFORMATION FOR SEQ ID NO: 12

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:12

AGUCAGAUCA UCUUCUCGAA CCCCGAGUGA CAA 33

(2) INFORMATION FOR SEQ ID NO: 13

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:13

UAGCCCAUGU UGUAGCAAAC CCUCAAGCUG AGGG 34

(2) INFORMATION FOR SEQ ID NO: 14

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:14

UACCUCAUCU ACUCC 15

(2) INFORMATION FOR SEQ ID NO: 15

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:15

UCAAGGGCCA AGGCUGCCCC UC 22

(2) INFORMATION FOR SEQ ID NO: 16

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:16

CACCCAUGUG CUCCUCACCC A 21

(2) INFORMATION FOR SEQ ID NO: 17

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:17

CGCAUCGCCG UCUCCUACCA GACCAA 26

(2) INFORMATION FOR SEQ ID NO: 18

(i) SEQUENCE CHARACTERISTICS:
(A) LENGTH: 11 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:18

GCCAUCAAGA G 11

(2) INFORMATION FOR SEQ ID NO: 19

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:19

CCGACUAUCU CGACUUUGCC 20

(2) INFORMATION FOR SEQ ID NO: 20

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:20

UCAUUGCCCU GUGAGGAGGA CGAACAUCCA ACCU 34

(2) INFORMATION FOR SEQ ID NO: 21

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:21

CUUAGGGUCG GAACCCAA 18

(2) INFORMATION FOR SEQ ID NO: 22

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:22

AAACCUGGGA UUCAGGAA 18

(2) INFORMATION FOR SEQ ID NO: 23

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:23

CUGGCAACCA CUAAGAAU 18

(2) INFORMATION FOR SEQ ID NO: 24

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:24

UCCAGAACUC ACUGG 15

(2) INFORMATION FOR SEQ ID NO: 25

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:25

UGGCCAGAAU GCUGCAGGAC UUGAGA 26

(2) INFORMATION FOR SEQ ID NO: 26

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:26

AGACCUCACC UAGAAAUUGA CACAAGU 27

(2) INFORMATION FOR SEQ ID NO: 27

(i) SEQUENCE CHARACTERISTICS:
(A) LENGTH: 11 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:27

CUUCCUUGAG A 11

(2) INFORMATION FOR SEQ ID NO: 28

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:28

UCUAUUUAUG UUUGCACUUG UG 22

(2) INFORMATION FOR SEQ ID NO: 29

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:29

AUUAUUUAUU AUUUAUUUAU UAUUUAUUUA UUUA 34

(2) INFORMATION FOR SEQ ID NO: 30

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:30

CAGAUGAAUG UAUUUAUUU 19

(2) INFORMATION FOR SEQ ID NO: 31

(i) SEQUENCE CHARACTERISTICS:
(A) LENGTH: 12 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:31

ACCGGGGUAU CC 12

(2) INFORMATION FOR SEQ ID NO: 32

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:32

GACCCAAUGU AGGAGCUGCC UUGGCUCAG 29

(2) INFORMATION FOR SEQ ID NO: 33

(i) SEQUENCE CHARACTERISTICS:
(A) LENGTH: 11 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:33

AGCCCCCUGG C 11

(2) INFORMATION FOR SEQ ID NO: 34

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:34

CCUUCUUUUG AUUAUGUUUU UUAAAAUAUU UAUC 34

(2) INFORMATION FOR SEQ ID NO: 35

(i) SEQUENCE CHARACTERISTICS:
(A) LENGTH: 14 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:35

GUCUAAACAA UGCU 14

(2) INFORMATION FOR SEQ ID NO: 36

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:36

GUCACUCAUU GCUGAG 16

(2) INFORMATION FOR SEQ ID NO: 37

(i) SEQUENCE CHARACTERISTICS:
(A) LENGTH: 12 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:37

CUACUAUUCA GU 12

(2) INFORMATION FOR SEQ ID NO: 38

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:38

GAAAUAAAGU UUGCUU 16

(2) INFORMATION FOR SEQ ID NO: 39

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:39

CUUUGCCAAA GGCAAAC 17

(2) INFORMATION FOR SEQ ID NO: 40

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:40

CGUUUCAGAG CCAUGAGGAU GC 22

(2) INFORMATION FOR SEQ ID NO: 41

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:41

AUUUGAGUUU GCUAGCUCUU GGAGCUG 27

(2) INFORMATION FOR SEQ ID NO: 42

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:42

CCUACGUGUA UGCCAUCC 18

(2) INFORMATION FOR SEQ ID NO: 43

(i) SEQUENCE CHARACTERISTICS:
(A) LENGTH: 14 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:43

AGACCUUGGC ACUG 14

(2) INFORMATION FOR SEQ ID NO: 44

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:44

UACUCAUCGA ACUCUGCUGA UA 22

(2) INFORMATION FOR SEQ ID NO: 45

(i) SEQUENCE CHARACTERISTICS:
(A) LENGTH: 12 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:45

UGUACAUAAA AA 12

(2) INFORMATION FOR SEQ ID NO: 46

(i) SEQUENCE CHARACTERISTICS:
(A) LENGTH: 11 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:46

AAACUGUGCA A 11

(2) INFORMATION FOR SEQ ID NO: 47

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:47

AAAGACUAUU CAAAAACUUG UCC 23

(2) INFORMATION FOR SEQ ID NO: 48

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:48

AGACGGAGAG UAAACCAAUU CCUAGACUAC CUGC 34

(2) INFORMATION FOR SEQ ID NO: 49

(i) SEQUENCE CHARACTERISTICS:
(A) LENGTH: 12 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:49

AAGAAAGAGU CA 12

(2) INFORMATION FOR SEQ ID NO: 50

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:50

ACUUCAGAGG GAAAG 15

(2) INFORMATION FOR SEQ ID NO: 51

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:51

AUUUCAGGCA UACUGACACU UUGCCAGAAA GCA 33

(2) INFORMATION FOR SEQ ID NO: 52

(i) SEQUENCE CHARACTERISTICS:
(A) LENGTH: 12 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:52

AUAUCAGAAU CA 12

(2) INFORMATION FOR SEQ ID NO: 53

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:53

CAAAAUUGAU AUACUUUUUU CUUAUUUAA 29

(2) INFORMATION FOR SEQ ID NO: 54

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:54

GAAAUGGUUA AGAAUUUG 18

(2) INFORMATION FOR SEQ ID NO: 55

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:55

UAGAGCUAAG UAAAGCCAG 19

(2) INFORMATION FOR SEQ ID NO: 56

(i) SEQUENCE CHARACTERISTICS:
(A) LENGTH: 13 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:56

ACACCAGCAU GAA 13

(2) INFORMATION FOR SEQ ID NO: 57

(i) SEQUENCE CHARACTERISTICS:
(A) LENGTH: 13 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:57

AGAAAUAUCU AGA 13

(2) INFORMATION FOR SEQ ID NO: 58

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:58

ACCUCAAAAA AGAUGUGAAA CAGU 24

(2) INFORMATION FOR SEQ ID NO: 59

(i) SEQUENCE CHARACTERISTICS:
(A) LENGTH: 14 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:59

AAAUGCAGAA GUUC 14

(2) INFORMATION FOR SEQ ID NO: 60

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:60

GACACUCUGG AGGUGAUGCG CAAGCCCAGG UGU 33

(2) INFORMATION FOR SEQ ID NO: 61

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:61

CUGAUGUUGG UCACUUCAGA AC 22

(2) INFORMATION FOR SEQ ID NO: 62

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:62

GCAUCCCGAA GUGGAGGAAA ACCCACCUUA CAU 33

(2) INFORMATION FOR SEQ ID NO: 63

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:63

AUUAUACACC AGAUUUGCCA AAAGAUG 27

(2) INFORMATION FOR SEQ ID NO: 64

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:64

CUGUUGAUUC UGCUGUUGAG A 21

(2) INFORMATION FOR SEQ ID NO: 65

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:65

CUGAAAGUCU GGGAAGAGGU GA 22

(2) INFORMATION FOR SEQ ID NO: 66

(i) SEQUENCE CHARACTERISTICS:
(A) LENGTH: 12 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:66

CUGAUAUAAU GA 12

(2) INFORMATION FOR SEQ ID NO: 67

(i) SEQUENCE CHARACTERISTICS:
(A) LENGTH: 12 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:67

UGCCUAUGCC CC 12

(2) INFORMATION FOR SEQ ID NO: 68

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:68

AUGCCCACUU UGAUGAUGAU GAACAAUGGA CA 32

(2) INFORMATION FOR SEQ ID NO: 69

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:69

AUUUCUCGUU GCUGCUCAUG 20

(2) INFORMATION FOR SEQ ID NO: 70

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:70

CACUCAGCCA ACACUGA 17

(2) INFORMATION FOR SEQ ID NO: 71

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:71

AAGAUGAUAU AAAUGGCAUU CAGUCC 26

(2) INFORMATION FOR SEQ ID NO: 72

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:72

CUCUAUGGAC CUCCCCCUGA CUCCCCU 27

(2) INFORMATION FOR SEQ ID NO: 73

(i) SEQUENCE CHARACTERISTICS:
(A) LENGTH: 14 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:73

CCCCCUGGUA CCCA 14

(2) INFORMATION FOR SEQ ID NO: 74

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:74

UCCUGCUUUG UCCUUUGAUG CUGUCAGCAC 30

(2) INFORMATION FOR SEQ ID NO: 75

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:75

AAUCCUGAUC UUUAAAGA 18

(2) INFORMATION FOR SEQ ID NO: 76

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:76

CAGGCACUUU UGGCGCAAAU CCC 23

(2) INFORMATION FOR SEQ ID NO: 77

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:77

AUUGCAUUUG AUCUCUUCAU UUUGGCCAUC 30

(2) INFORMATION FOR SEQ ID NO: 78

(i) SEQUENCE CHARACTERISTICS:
(A) LENGTH: 13 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:78

GCAUAUGAAG UUA 13

(2) INFORMATION FOR SEQ ID NO: 79

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:79

AAAUCGAUGC AGCCAUUUCU GA 22

(2) INFORMATION FOR SEQ ID NO: 80

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:80

UUUGAUGAGA AGAGAAAUUC CAUGGAGC 28

(2) INFORMATION FOR SEQ ID NO: 81

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:81

CAGGCUUUCC CAAGCAAAUA GCUGAAGAC 29

(2) INFORMATION FOR SEQ ID NO: 82

(i) SEQUENCE CHARACTERISTICS:
(A) LENGTH: 13 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:82

CCCAAAUGCA AAG 13

(2) INFORMATION FOR SEQ ID NO: 83

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:83

AUGUAGAAGG CACAAUAUGG GCACUUUAAA 30

(2) INFORMATION FOR SEQ ID NO: 84

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:84

UCUUGCCGGU CAUUUUUAUG UUAU 24

(2) INFORMATION FOR SEQ ID NO: 85

(i) SEQUENCE CHARACTERISTICS:
(A) LENGTH: 13 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:85

GCUGCUGCUU AGC 13

(2) INFORMATION FOR SEQ ID NO: 86

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:86

CAACAGACAA GUGACUGUAU CU 22

(2) INFORMATION FOR SEQ ID NO: 87

(i) SEQUENCE CHARACTERISTICS:
(A) LENGTH: 11 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:87

CUUAUUUAAU A 11

(2) INFORMATION FOR SEQ ID NO: 88

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:88

GGCGGCAGCG CCCUGCCGAC GCCGGGG 27

(2) INFORMATION FOR SEQ ID NO: 89

(i) SEQUENCE CHARACTERISTICS:
(A) LENGTH: 14 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:89

CCGCGGCUCU CGGC 14

(2) INFORMATION FOR SEQ ID NO: 90

(i) SEQUENCE CHARACTERISTICS:
(A) LENGTH: 13 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:90

GCCAUGGCCC GAA 13

(2) INFORMATION FOR SEQ ID NO: 91

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:91

CGGCACAGCA UAUAUAGCAG UGACGAGGA 29

(2) INFORMATION FOR SEQ ID NO: 92

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:92

GACUUUGAGA UGUGUGACCA UGACUAUGAU GGG 33

(2) INFORMATION FOR SEQ ID NO: 93

(i) SEQUENCE CHARACTERISTICS:
(A) LENGTH: 12 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:93

CUGGAAAGCG UC 12

(2) INFORMATION FOR SEQ ID NO: 94

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:94

GGAAGAGGAU GAAAAACUGA AGAAG 25

(2) INFORMATION FOR SEQ ID NO: 95

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:95

GAAGAACUGG UGGAACAGAA UGGAAC 26

(2) INFORMATION FOR SEQ ID NO: 96

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:96

CUGGAAAGUU AUUGCCAA 18

(2) INFORMATION FOR SEQ ID NO: 97

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:97

CCCGAAUCGA ACAGAUGUGC AG 22

(2) INFORMATION FOR SEQ ID NO: 98

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:98

GAAAGUACUA AACCCUGAG 19

(2) INFORMATION FOR SEQ ID NO: 99

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:99

CUUGGACCAA AGAAGAAGAU CAGAGAGUGA UA 32

(2) INFORMATION FOR SEQ ID NO: 100

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:100

ACAGAAAUAC GGUCCGAAAC GUUGGUCUG 29

(2) INFORMATION FOR SEQ ID NO: 101

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:101

UUAUUGCCAA GCACUUAAAG GGGAGAAUUG GAA 33

(2) INFORMATION FOR SEQ ID NO: 102

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:102

GAAUCCAGAA GUUAAGAA 18

(2) INFORMATION FOR SEQ ID NO: 103

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:103

GGAAGACAGA AUUAUUUACC AGGCACA 27

(2) INFORMATION FOR SEQ ID NO: 104

(i) SEQUENCE CHARACTERISTICS:
(A) LENGTH: 20 base pairs
(B) TYPE: nucleic acid
(C) STRANDEDNESS: single
DD) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:104

CAAGAGACUG GGGAACAGAU 20

(2) INFORMATION FOR SEQ ID NO: 105

(i) SEQUENCE CHARACTERISTICS:
(A) LENGTH: 14 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:105

AAAUCGCAAA GCUA 14

(2) INFORMATION FOR SEQ ID NO: 106

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:106

GGACGAACUG AUAAUGCUAU CAAGAACC 28

(2) INFORMATION FOR SEQ ID NO: 107

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:107

ACUGGAAUUC UACAAUGCGU CGGAAGGUCG AACA 34

(2) INFORMATION FOR SEQ ID NO: 108

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:108

CAGCCAGCAG UGGCCACAA 19

(2) INFORMATION FOR SEQ ID NO: 109

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:109

CAUUUGAUGG GUUUUGCUCA GGCUCCGCCU ACA 33

(2) INFORMATION FOR SEQ ID NO: 110

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:110

GCUCAACUCC CUGCCACUGG CCAGCCC 27

(2) INFORMATION FOR SEQ ID NO: 111

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:111

AACAACGACU AUUCCUAUUA CCACA 25

(2) INFORMATION FOR SEQ ID NO: 112

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:112

CAAAAUGUCU CCAGUCAUGU UCCAUACCCU 30

(2) INFORMATION FOR SEQ ID NO: 113

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:113

AAAUAUAGUC AAUGUCCCUC AGCCAGCUGC CGCA 34

(2) INFORMATION FOR SEQ ID NO: 114

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:114

AGAGACACUA UAAUGAUGAA GACCCUGAGA AGGA 34

(2) INFORMATION FOR SEQ ID NO: 115

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:115

AAAGCGAAUA AAGGAAUUAG AAUUG 25

(2) INFORMATION FOR SEQ ID NO: 116

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:116

CGGUGUACUU ACUGCC 16

(2) INFORMATION FOR SEQ ID NO: 117

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:117

AGCUAAAAGG ACAGCAGGUG CUACCAACAC AGAA 34

(2) INFORMATION FOR SEQ ID NO: 118

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:118

CCCGGGUGGC ACAGCACCAC CAUUGCCGAC CACA 34

(2) INFORMATION FOR SEQ ID NO: 119

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:119

AACACCACUC CACUCCAUCU CUGCCAGCGG AUCC 34

(2) INFORMATION FOR SEQ ID NO: 120

(i) SEQUENCE CHARACTERISTICS:
(A) LENGTH: 12 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:120

UACCUGAAGA AA 12

(2) INFORMATION FOR SEQ ID NO: 121

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:121

AUGAUCGUCC ACCAGGGCAC CAUU 24

(2) INFORMATION FOR SEQ ID NO: 122

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:122

CAGAAACACU CCAAUUUA 18

(2) INFORMATION FOR SEQ ID NO: 123

(i) SEQUENCE CHARACTERISTICS:
(A) LENGTH: 11 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:123

AAACUCAGAC U 11

(2) INFORMATION FOR SEQ ID NO: 124

(i) SEQUENCE CHARACTERISTICS:
(A) LENGTH: 14 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:124

AUGCCUUCUU UAAC 14

(2) INFORMATION FOR SEQ ID NO: 125

(i) SEQUENCE CHARACTERISTICS:
(A) LENGTH: 11 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:125

UUACAACACC A 11

(2) INFORMATION FOR SEQ ID NO: 126

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:126

ACUCAAAAGG AAAAUACUGU UUUUAGAACC C 31

(2) INFORMATION FOR SEQ ID NO: 127

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:127

CAGCUAUCAA AAGGUCAAUC UUAGAAAGCU 30

(2) INFORMATION FOR SEQ ID NO: 128

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:128

CUCCAAGAAC UCCUACACCA UUCAA 25

(2) INFORMATION FOR SEQ ID NO: 129

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:129

ACAUGCACUU GCAGCUCAAG AA 22

(2) INFORMATION FOR SEQ ID NO: 130

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:130

UACGGUCCCC UGAAGAUGCU ACCUCAGA 28

(2) INFORMATION FOR SEQ ID NO: 131

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:131

CACCCUCUCA UCUAGUAGAA GAUCUGCAGG A 31

(2) INFORMATION FOR SEQ ID NO: 132

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:132

UCAAACAGGA AUCUGAUGAA UCUGGA 26

(2) INFORMATION FOR SEQ ID NO: 133

(i) SEQUENCE CHARACTERISTICS:
(A) LENGTH: 11 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:133

AAGAAAAUGG A 11

(2) INFORMATION FOR SEQ ID NO: 134

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:134

CUUACUGAAG AAAAUCAAAC AAGA 24

(2) INFORMATION FOR SEQ ID NO: 135

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:135

AAUCUCCAAC UGAUAAAUCA G 21

(2) INFORMATION FOR SEQ ID NO: 136

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:136

GCUCACACCA CUGGGA 16

(2) INFORMATION FOR SEQ ID NO: 137

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:137

CCUCGCCUGU GCGAGAUGCA CCGAAUAUUC 30

(2) INFORMATION FOR SEQ ID NO: 138

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:138

GGCACCAGCA UCAGAAGAUG AAGAC 25

(2) INFORMATION FOR SEQ ID NO: 139

(i) SEQUENCE CHARACTERISTICS:
(A) LENGTH: 13 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:139

CAUUUACAGU ACC 13

(2) INFORMATION FOR SEQ ID NO: 140

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:140

CCCUGGCGAG CCCCUUGCA 19

(2) INFORMATION FOR SEQ ID NO: 141

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:141

GCCUUGUAGC AGUACCUGGG A 21

(2) INFORMATION FOR SEQ ID NO: 142

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:142

GUCAAGCUCG UAAAUACGUG AA 22

(2) INFORMATION FOR SEQ ID NO: 143

(i) SEQUENCE CHARACTERISTICS:
(A) LENGTH: 11 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:143

GAACAGUUCA A 11

(2) INFORMATION FOR SEQ ID NO: 144

(i) SEQUENCE CHARACTERISTICS:
(A) LENGTH: 14 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:144

AUGAAACUUU UCAU 14

(2) INFORMATION FOR SEQ ID NO: 145

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:145

AAAAUAAAUA ACAGUC 16

(2) INFORMATION FOR SEQ ID NO: 146

(i) SEQUENCE CHARACTERISTICS:
(A) LENGTH: 12 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:146

UGAAUUGUAG CC 12

(2) INFORMATION FOR SEQ ID NO: 147

(i) SEQUENCE CHARACTERISTICS:
(A) LENGTH: 13 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:147

UUAAUAUCUU AAU 13

(2) INFORMATION FOR SEQ ID NO: 148

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:14

AUUUAUCUGG UAUUUUAAAG GAUCCAACAG AUC 33

(2) INFORMATION FOR SEQ ID NO: 149

(i) SEQUENCE CHARACTERISTICS:
(A) LENGTH: 11 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:149

CCAGUAUUUC A 11

(2). INFORMATION FOR SEQ ID NO: 150

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:150

CUCGAUCACU AAACAUAUG 19

(2) INFORMATION FOR SEQ ID NO: 151

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:151

CAUAUAUUUU UAAAAAUC 18

(2) INFORMATION FOR SEQ ID NO: 152

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:152

UGCUAUGGUC UUAGCCU 17

(2) INFORMATION FOR SEQ ID NO: 153

(i) SEQUENCE CHARACTERISTICS:
(A) LENGTH: 11 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:153

AGUAUCAGAG G 11

(2) INFORMATION FOR SEQ ID NO: 154

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:154

UAGGUAAUUG ACUAU 15

(2) INFORMATION FOR SEQ ID NO: 155

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:155

UAUUUCAGAC UUUUUAAUUU UAUAUAUAUA UACA 34

(2) INFORMATION FOR SEQ ID NO: 156

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:156

CAAUACAUUU GAAAACUUGU UUGGGAGACU CUGC 34

(2) INFORMATION FOR SEQ ID NO: 157

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:157

GUGGUUUUUU UGUUAUUGUU GGUUU 25

(2) INFORMATION FOR SEQ ID NO: 158

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:158

UUCUUUUUUG GGAGAU 16

(2) INFORMATION FOR SEQ ID NO: 159

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:159

CUAUGUUUUG UUUUG 15

(2) INFORMATION FOR SEQ ID NO: 160

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:160

AGCCUGACUG UUUUAUA 17

(2) INFORMATION FOR SEQ ID NO: 161

(i) SEQUENCE CHARACTERISTICS:
(A) LENGTH: 11 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:161

UCGAUUUGAU C 11

(2) INFORMATION FOR SEQ ID NO: 162

(i) SEQUENCE CHARACTERISTICS:
(A) LENGTH: 13 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:162

UGGAUCCUGU GUU 13

(2) INFORMATION FOR SEQ ID NO: 163

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:163

UUGAUAGCCA GUCACUGCCU UAAGA 25

(2) INFORMATION FOR SEQ ID NO: 164

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:164

ACAUUUGAUG CAAGAUGGCC AGCACU 26

(2) INFORMATION FOR SEQ ID NO: 165

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:165

GCUACUGCAG GACUUCCCAG C 21

(2) INFORMATION FOR SEQ ID NO: 166

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:166

CUCCUCUUCC UGCUGCUCGC UAGG 24

(2) INFORMATION FOR SEQ ID NO: 167

(i) SEQUENCE CHARACTERISTICS:
(A) LENGTH: 13 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:167

GCCAGGAGGC AUC 13

(2) INFORMATION FOR SEQ ID NO: 168

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:168

AACAGGUGAC AGUCACCCAU G 21

(2) INFORMATION FOR SEQ ID NO: 169

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:169

CCCAGAGCCC AAACCUGGUG A 21

(2) INFORMATION FOR SEQ ID NO: 170

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:170

CAGCAAGUUU GUGGAGGAAU AUGA 24

(2) INFORMATION FOR SEQ ID NO: 171

(i) SEQUENCE CHARACTERISTICS:
(A) LENGTH: 12 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:171

CCGGACAUCC CA 12

(2) INFORMATION FOR SEQ ID NO: 172

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:172

GAACGAGUAU GCCGAGGCC 19

(2) INFORMATION FOR SEQ ID NO: 173

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:173

AACUGGAACU ACAACAC 17

(2) INFORMATION FOR SEQ ID NO: 174

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:174

GAGACCAGCA AGAUUCUGCU G 21

(2) INFORMATION FOR SEQ ID NO: 175

(i) SEQUENCE CHARACTERISTICS:
(A) LENGTH: 12 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:175

ACAUGCAAAU AG 12

(2) INFORMATION FOR SEQ ID NO: 176

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:176

ACACCCUGAA GUACGGCACC CAG 23

(2) INFORMATION FOR SEQ ID NO: 177

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:177

GUGAACCAGU UGCAGAACAC CACUA 25

(2) INFORMATION FOR SEQ ID NO: 178

(i) SEQUENCE CHARACTERISTICS:
(A) LENGTH: 11 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:178

AGGACCUAGA A 11

(2) INFORMATION FOR SEQ ID NO: 179

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:179

GCGCUGCCUG CCCAGGAGCU GGAG 24

(2) INFORMATION FOR SEQ ID NO: 180

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:180

GAGUACAACA AGAUCCUGUU GGA 23

(2) INFORMATION FOR SEQ ID NO: 181

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:181

GGAUAUGGAA ACCACCUACA GC 22

(2) INFORMATION FOR SEQ ID NO: 182

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:182

CUGUGUGCCA CCCGAAUGGC 20

(2) INFORMATION FOR SEQ ID NO: 183

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:183

CGAGCCAGAU CUGACGAAUG UGAUG 25

(2) INFORMATION FOR SEQ ID NO: 184

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:184

CAUCCCGGAA AUAUGAAGAC CUG 23

(2) INFORMATION FOR SEQ ID NO: 185

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:185

CCUGUUAUGG GCAUGGG 17

(2) INFORMATION FOR SEQ ID NO: 186

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:186

CUGGCGAGAC AAGGCGGG 18

(2) INFORMATION FOR SEQ ID NO: 187

(i) SEQUENCE CHARACTERISTICS:
(A) LENGTH: 11 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:187

CCCGAAAUAC G 11

(2) INFORMATION FOR SEQ ID NO: 188

(i) SEQUENCE CHARACTERISTICS:
(A) LENGTH: 10 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:188

AUCAACCAGG 10

(2) INFORMATION FOR SEQ ID NO: 189

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:189

GUAGAUGCAG GGGACUC 17

(2) INFORMATION FOR SEQ ID NO: 190

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:190

AGGUCUAUGU ACGAGACACC AUCC 24

(2) INFORMATION FOR SEQ ID NO: 191

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:191

AGCUGCAGCC ACUCUACCUC AAC 23

(2) INFORMATION FOR SEQ ID NO: 192

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:192

CUGCAUGCCU ACGUGCGCCG 20

(2) INFORMATION FOR SEQ ID NO: 193

(i) SEQUENCE CHARACTERISTICS:
(A) LENGTH: 10 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:193

CAGCAUCAAC 10

(2) INFORMATION FOR SEQ ID NO: 194

(i) SEQUENCE CHARACTERISTICS:
(A) LENGTH: 14 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:194

CCAUUCCUGC UCAC 14

(2) INFORMATION FOR SEQ ID NO: 195

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:195

CAGACCUGGU CCAAC 15

(2) INFORMATION FOR SEQ ID NO: 196

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:196

AUCUAUGACU UGGUGG
16

(2) INFORMATION FOR SEQ ID NO: 197

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:197

CUUCAGCCCC CUCGAUGGAC 20

(2) INFORMATION FOR SEQ ID NO: 198

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:198

CACCACAGAG GCUAUGCUAA 20

(2) INFORMATION FOR SEQ ID NO: 199

(i) SEQUENCE CHARACTERISTICS:
(A) LENGTH: 10 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:199

GGCUGGACGC 10

(2) INFORMATION FOR SEQ ID NO: 200

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:200

GAGGAUGUUU AAGGAGGCUG AUGA 24

(2) INFORMATION FOR SEQ ID NO: 201

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:201

CUGAUGAUUU CUUCACCUCC 20

(2) INFORMATION FOR SEQ ID NO: 202

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:202

UGCCUCCUGA GUUCUGGAAC A 21

(2) INFORMATION FOR SEQ ID NO: 203

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:203

AAGUCGAUGC UGGAGAAG 18

(2) INFORMATION FOR SEQ ID NO: 204

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:204

ACGCCUCGGC CUGGGACUUC UACAA 25

(2) INFORMATION FOR SEQ ID NO: 205

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:205

AGGACUUCCG GAUCAAGCAG UGCA 24

(2) INFORMATION FOR SEQ ID NO: 206

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:206

CCACCGUGAA CUUGGAGGAC CUGG 24

(2) INFORMATION FOR SEQ ID NO: 207

(i) SEQUENCE CHARACTERISTICS:
(A) LENGTH: 14 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:207

ACAUCCAGUA UUUC 14

(2) INFORMATION FOR SEQ ID NO: 208

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:208

GCAGUACAAA GACUUACCUG UGG 23

(2) INFORMATION FOR SEQ ID NO: 209

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:209

CUUGAGGGAG GGUGCCAACC 20

(2) INFORMATION FOR SEQ ID NO: 210

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:210

CCGGCUUCCA UGAGGCCAUU GG 22

(2) INFORMATION FOR SEQ ID NO: 211

(i) SEQUENCE CHARACTERISTICS:
(A) LENGTH: 10 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:211

GACGUGCUAG 10

(2) INFORMATION FOR SEQ ID NO: 212

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:212

GUGUCUACGC CCAAGCACCU GCACA 25

(2) INFORMATION FOR SEQ ID NO: 213

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:213

GUCUCAACCU GCUGAGCAG 19

(2) INFORMATION FOR SEQ ID NO: 214

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:214

CAGCGACGAG CAUGACAUCA AC 22

(2) INFORMATION FOR SEQ ID NO: 215

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:215

CUUUCUGAUG AAGAUGGCCC UUG 23

(2) INFORMATION FOR SEQ ID NO: 216

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:216

CUUGACAAGA UCGCC 15

(2) INFORMATION FOR SEQ ID NO: 217

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:217

ACCUCGUCGA UCAGUGGCG 19

(2) INFORMATION FOR SEQ ID NO: 218

(i) SEQUENCE CHARACTERISTICS:
(A) LENGTH: 11 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:218

GAAGCAUCAC C 11

(2) INFORMATION FOR SEQ ID NO: 219

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:219

AACUAUAACC AGGAGUGG 18

(2) INFORMATION FOR SEQ ID NO: 220

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:220

GCCUCAGGCU GAAGUA 16

(2) INFORMATION FOR SEQ ID NO: 221

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:221

CCAGGGCCUC UGCCCCCCAG 20

(2) INFORMATION FOR SEQ ID NO: 222

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:222

AGGACUCAAG GUGAC 15

(2) INFORMATION FOR SEQ ID NO: 223

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:223

CUUUGACCCA GGGGCC 16

(2) INFORMATION FOR SEQ ID NO: 224

(i) SEQUENCE CHARACTERISTICS:
(A) LENGTH: 14 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:224

CUAGCGUGCC UUAC 14

(2) INFORMATION FOR SEQ ID NO: 225

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:225

CAUCCAGUUC CAGUUCCACG 20

(2) INFORMATION FOR SEQ ID NO: 226

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:226

GUUCCACGAG GCACUG 16

(2) INFORMATION FOR SEQ ID NO: 227

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:227

GCCCCCUGCA CAAGUGUGAC AUC 23

(2) INFORMATION FOR SEQ ID NO: 228

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:228

CUACCAGUCC AAGGAG 16

(2) INFORMATION FOR SEQ ID NO: 229

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:229

GAGCUACUUC AAGCUGCUGG 20

(2) INFORMATION FOR SEQ ID NO: 230

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:230

GGACUGGCUC CGCACGG 17

(2) INFORMATION FOR SEQ ID NO: 231

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:231

AGUACAACUG GACGCC 16

(2) INFORMATION FOR SEQ ID NO: 232

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:232

GAACUCCGCU CGCUCAGAAG G 21

(2) INFORMATION FOR SEQ ID NO: 233

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:233

GCCCCUCCCA GACAG 15

(2) INFORMATION FOR SEQ ID NO: 234

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:234

ACCUGGAUGC GCAGCA 16

(2) INFORMATION FOR SEQ ID NO: 235

(i) SEQUENCE CHARACTERISTICS:
(A) LENGTH: 12 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:235

AGUGGCUGCU GC 12

(2) INFORMATION FOR SEQ ID NO: 236

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:236

CGCCCUGCUG GUAGCCACCC 20

(2) INFORMATION FOR SEQ ID NO: 237

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:237

AUCCGCCACC GCAGCCUCC 19

(2) INFORMATION FOR SEQ ID NO: 238

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:238

ACACUCCUGA GGUGACCCGG 20

(2) INFORMATION FOR SEQ ID NO: 239

(i) SEQUENCE CHARACTERISTICS:
(A) LENGTH: 11 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:239

GCCCACCCUG C 11

(2) INFORMATION FOR SEQ ID NO: 240

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:240

CUGUCCCUGU CCCCCUCCCC 20

(2) INFORMATION FOR SEQ ID NO: 241

(i) SEQUENCE CHARACTERISTICS:
(A) LENGTH: 12 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:241

CUCCAGACCA CC 12

(2) INFORMATION FOR SEQ ID NO: 242

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:242

AGCCCCUUCU CCCAGCACAC 20

(2) INFORMATION FOR SEQ ID NO: 243

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:243

CUGCCUGACA CUGAGCCC 18

(2) INFORMATION FOR SEQ ID NO: 244

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:244

CACCUCUCCA AGUCUCUCUG 20

(2) INFORMATION FOR SEQ ID NO: 245

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:245

UGAAUACAAU UAAAGGUCCU G 21

□

## Claims

1. An enzymatic RNA molecule which cleaves mRNA produced from a gene encoding NF-κB.

2. The enzymatic RNA molecule of claim 1 wherein said RNA molecule is in a hammerhead motif.

3. The enzymatic RNA molecule of claim 1 wherein said RNA molecule is in a hairpin, hepatitis Delta virus, group 1 intron, or RNaseP RNA motif.

4. The enzymatic RNA molecule of claim 2 wherein said ribozyme comprises between 5 and 23 bases complementary to said mRNA.

5. The enzymatic RNA molecule of claim 4, wherein said ribozyme comprises between 10 and 18 bases complementary to said mRNA.

6. A mammalian cell including an enzymatic RNA molecule of claim 1.

7. The cell of claim 6, wherein said cell is a human cell.

8. An expression vector including nucleic acid encoding the enzymatic RNA molecule of claim 1, in a manner which allows expression of that enzymatic RNA molecule within a mammalian cell.

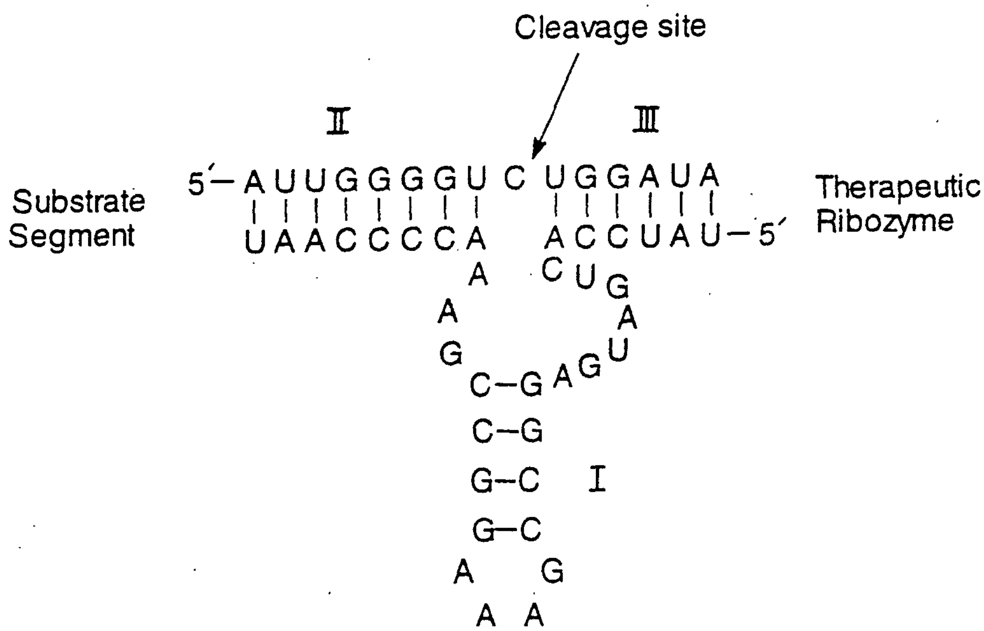
Cleavage site
II
III
Substrate Segment
Therapeutic Ribozyme
5′–AUUGGGGU C UGGAUA
UAACCCCA ACCUAU–5′
A
A
G
CUG
A
U
C–G AG
C–G
G–C I
G–C
A G
A A

FIG. 1